# EUROPÄISCHE PATENTANMELDUNG

(11) **EP 2 158 896 A2**
(43) Veröffentlichungstag der Anmeldung: **03.03.2010**
(21) Anmeldenummer: 09174780.8
(22) Anmeldetag: 02.11.2009
(51) Int. Cl.: A61K 8/34, A61K 8/37, A61Q 5/06, A61Q 15/00, A61Q 17/04, A61Q 19/00, A61Q 19/10, C11B 9/00

(54) **Riechstoffhaltige Zusammensetzungen umfassend Neopentylglycoldiisononanoat**

(71) Anmelder: Symrise GmbH & Co. KG, 37603 Holzminden (DE)
(72) Erfinder: Wöhrle, Ingo, 28203, Bremen (DE); Koch, Oskar, 37079, Göttingen (DE); Meier, Manfred, 37699, Fürstenberg (DE); Schmaus, Gerhard, 37671, Höxter-Bosseborn (DE)
(74) Vertreter: Eisenführ, Speiser & Partner

(57) **Zusammenfassung**

Die Erfindung betrifft die Verwendung von Neopentylglycoldüsononanoat (CAS Nummer 27841-07-2) als Fixateur für Parfümöle bzw. als Fixateur für (Kopfnoten-) Riechstoffe. Ferner betrifft die Erfindung bestimmte Zusammensetzungen enthaltend Neopentylglycoldüsononanoat sowie einen oder mehrere Kopfnoten-Riechstoffe. Die Erfindung betrifft ferner ein Verfahren zur Herstellung solcher Zusammensetzungen sowie (kosmetische) Produkte enthaltend eine erfindungsgemäße Zusammensetzung. Ferner betrifft die Erfindung ein Verfahren zur Vermittlung, Verstärkung oder Modifizierung eines Geruchs auf der (menschlichen) Haut. Ein besonderer Aspekt der vorliegenden Erfindung betrifft die Verbesserung der Haftfestigkeit von leichtflüchtigen Riechstoffen auf (menschlicher) Haut und/oder (menschlichem) Haar. Daneben betrifft die Erfindung eine Zusammenstellung (Kit) umfassend Neopentylglycoldüsononanoat und einen oder mehrere Kopfnoten-Riechstoffe.

## Beschreibung

Die Erfindung betrifft die Verwendung von Neopentylglycoldiisononanoat (CAS Nummer 27841-07-2) als Fixateur für Parfümöle bzw. als Fixateur für (Kopfnoten-) Riechstoffe. Ferner betrifft die Erfindung bestimmte Zusammensetzungen enthaltend Neopentylglycoldiisononanoat sowie einen oder mehrere Kopfnoten-Riechstoffe. Die Erfindung betrifft ferner ein Verfahren zur Herstellung solcher Zusammensetzungen sowie (kosmetische) Produkte enthaltend eine erfindungsgemäße Zusammensetzung. Ferner betrifft die Erfindung ein Verfahren zur Vermittlung, Verstärkung oder Modifizierung eines Geruchs auf der (menschlichen) Haut. Ein besonderer Aspekt der vorliegenden Erfindung betrifft die Verbesserung der Haftfestigkeit von leichtflüchtigen Riechstoffen auf (menschlicher) Haut und/oder (menschlichem) Haar. Daneben betrifft die Erfindung eine Zusammenstellung (Kit) umfassend Neopentylglycoldiisononanoat und einen oder mehrere Kopfnoten-Riechstoffe.

Im Bereich der Riechstoffe und der Parfümerie ist es allgemein bekannt, dass es bei Anwendung von Riechstoffzusammensetzungen auf der Haut durch die Verdampfung von Verdünnungs- oder Lösemitteln zu einer Abnahme von Riechstoffen, insbesondere der leichtflüchtigen Kopfnote (Topnote), kommt, während die schwerer flüchtigen Noten, insbesondere die sogenannten Fondnoten, eine hervorragende Haftung auf der Haut aufweisen und über einen langen Zeitraum von der Haut abgegeben und dementsprechend geruchlich länger wahrgenommen werden. Durch die schnellere Verdampfung der Kopfnote kommt es folglich im zeitlichen Verlauf zu einer deutlichen Veränderung des Geruchsprofils von auf Haut aufgetragenen Riechstoffzusammensetzungen. Ein ähnlicher Effekt tritt bei der Anwendung Riechstoffzusammensetzungen auf (menschlichem) Haar auf. Hierbei ist zu berücksichtigen, dass der beschriebene nachteilige Effekt insbesondere dann als problematisch empfunden wird, wenn eine Riechstoffzusammensetzung (Riechstoffmischung) als leave-on-Produkt eingesetzt wird, das heißt auf der Haut und/oder dem Haar verbleiben soll, um von dort aus über einen längeren Zeitraum abgegeben zu werden.

Unter Haftung und Haftfestigkeit wird im Rahmen des vorliegenden Textes die Haftung eines Riechstoffes auf Haut und Haar, insbesondere die Haftung auf menschlicher Haut (ausschließlich der (Mund)Schleimhaut) verstanden. Wenngleich also die vorliegende Erfindung hinsichtlich der Applikation von Riechstoffzusammensetzungen auch die (Mund)Schleimhaut betrifft, ist doch festzustellen, dass die Bedeutung der vorliegenden Erfindung hinsichtlich der Aspekte Haut (ausschließlich (Mund)Schleimhaut) und/oder Haar (insbesondere menschliche Haut) von noch weit höherer Relevanz ist, denn bei Applikation einer Riechzusammensetzung auf die (Mund)Schleimhaut wird regelmäßig der negative Verdampfungseffekt überlagert und dominiert von Abwaschungseffekten (im Bereich der Mundschleimhaut beispielsweise unterstützt durch den Einfluss von Speichel).

In der parfümistischen Praxis wurden bereits zahlreiche Versuche unternommen, um die Haftfestigkeit bzw. die Wahrnehmbarkeit, insbesondere der Kopfnote, von Riechstoffzusammensetzungen, dabei insbesondere auf menschlicher Haut, zu verlängern und somit eine gewisse geruchliche "Profilstabilität" zu erreichen.

Ein Fixateur erhöht die Haftfestigkeit von Riechstoffen, z.B. durch deren Dampfdruckerniedrigung. Unter Fixateuren versteht man hierbei solche Stoffe, die eine zeitlich verzögerte Freisetzung der Parfümölbestandteile, beispielsweise auf Haut und/oder Haar, ermöglichen und somit einen länger anhaltenden Dufteindruck gewährleisten.

Besonders geeignet sind hierbei solche Fixateure, die geruchlos sind oder über einen sehr geringen Eigengeruch verfügen, so dass sie den Geruchseindruck von Riechstoffen, Riechstoffmischungen und Parfümölen nicht verändern.

Die Verwendung von Fixateuren ist vielfach beschrieben, d.h. die Verwendung von Einzelstoffen oder Kombinationen von (Riech)Stoffen, die leichter flüchtigen Riechstoffen oder Riechstoffmischungen zugesetzt werden, um deren Verdampfungsgeschwindigkeit zu reduzieren (vgl. US 6,737,396).

In IP.COM #000033581D (veröffentlicht Januar 2005) wird die Verwendung von Hydroxyalkylharnstoff-Derivaten, insbesondere Hydroxyethylharnstoff, beschrieben, wodurch die Haftungseigenschaften von Parfüms in kosmetischen Anwendungen, insbesondere wässrig-ethanolischen Formulierungen, auf Haut und Haar verlängert wird.

In US 3,939,099 wird die Verwendung von Filmbildnern beschrieben, die sich in einem Wasser/Ethanolgemisch lösen und mit Riechstoff(mischungen) mischbar sind. Als Beispiele werden ionische und nichtionische Derivate von wasserlöslichen Polymeren genannt wie z.B. Polyvinylpyrrolidonderivate, quarternäre Polyvinylpyrrolidone mit Molgewichten im Bereich 50000 - 1000000, kationische Cellulosederivate und ähnliche. Während des Verdampfens von Ethanol kommt es zur Bildung eines Films auf der Haut, in den die Riechstoffe eingelagert sind.

US 6,210,688 beschreibt die Bildung und Verwendung eines geruchlosen Polymerfilms auf der Haut (basierend auf Vinylether-Copolymeren, Polyacrylaten, Methacrylaten, Polyestern, Polyfluorkohlenwasserstoffen, Polysaccariden), auf den anschließend ein Parfüm appliziert wird. Dadurch sollen "Reaktionen" mit der Haut unterbunden werden.

FR 2 747 306 beschreibt die Verwendung polymerer Kohlenwasserstoffe (Polyethylene mit Molekulargewichten zwischen 3000 und 30000). Da diese Polymere in Ethanol/Wasser unlöslich sind, ist ein entsprechendes Produkt wie z.B. Eau de Cologne, EdT oder ein Eau de Parfum trübe.

WO 2004/098556 beschreibt eine neuartige sprühbare und klare Parfümformulierung, die sich durch eine erhöhte Oberflächenspannung bzw. verringerte Berührungsfläche nach Applikation auszeichnet, die durch die Anwendung einer wirksamen Menge eines Polymers erreicht wird. Durch die kleinere Berührungsfläche wird das verbleibende Parfümöl nach dem Verdampfen des Ethanols auf einer kleineren Fläche konzentriert, von der die Riechstoffe langsamer abdampfen.

Filmbildner und/oder Polymere haben den Nachteil, dass sie nicht nur die Verdampfungsgeschwindigkeit der Kopfnote, sondern auch die aller anderen, schwerer flüchtigen Riechstoffe reduzieren, wodurch die Gesamtintensität merklich reduziert wird. Weiterhin können Filmbildner ein unangenehmes klebriges bzw. spannendes Hautgefühl hervorrufen.

EP 0 181 401 und EP 0 857 481 beschreiben gelartige Parfümzubereitungen, in denen die Diffusion und damit auch die Verdampfung von Riechstoffen reduziert wird. Die vorgeschlagene Gelbildung ist allerdings insbesondere für manche Anwendungsbereiche, wie beispielsweise der Feinparfümerie oder Produkten in Form von Lotionen oder Sprays nicht geeignet, da die dort beschriebenen gelartigen Zubereitungen zu viskos sind.

EP 1 872 831 beschreibt bestimmte ethanolische Riechstoffzusammensetzungen enthaltend deliqueszente Stoffe zur Vermittlung, Verstärkung oder Modifizierung eines Geruchs, dabei insbesondere die Verbesserung der Haftfestigkeit von ethanolischen Riechstoffzusammensetzungen auf (menschlicher) Haut und/oder (menschlichem) Haar.

Die gesuchten Fixateure sollen den obigen Anforderungen gerecht werden und mit möglichst vielen gängigen (Parfümerie-)Riechstoffen und Parfümölen gut mischbar und darüber hinaus in eine Vielzahl kosmetischer Formulierungen eingearbeitet werden können.

Es war daher die primäre Aufgabe der vorliegenden Erfindung, eine (alternative) Riechstoffzusammensetzung anzugeben, in der die eingesetzten Riechstoffe, vorzugsweise Riechstoffe, welche die Kopfnote eines Duftes bilden, eine lange (gegenüber konventionellen Riechstoffzusammensetzungen verlängerte) Haftung (Haftfestigkeit), insbesondere auf (menschlicher) Haut (damit ist Haut exklusive Schleimhaut gemeint) und/oder (menschlichem) Haar besitzen. Dabei sollten die vorstehend geschilderten Nachteile bisheriger Riechstoffzusammensetzungen möglichst vermieden werden.

Die primäre gestellte Aufgabe wird erfindungsgemäß gelöst durch eine Zusammensetzung umfassend (oder bestehend aus):
(A) Neopentylglycoldiisononanoat,
   und
(B) einem oder mehreren Riechstoffen, vorzugsweise einer Riechstoffmischung umfassend 2, 3, 4, 5, 6, 7, 8, 9, 10 oder mehr Riechstoffe,
wobei Bestandteil (B)
(B) (i) einen, 2, 3, 4, 5 oder mehr Riechstoffe mit einem Molgewicht im Bereich von 100 g/mol bis 175 g/mol (Kopfnote, Topnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
und
wobei Bestandteil (A) vorzugsweise in einer für den, mehrere oder sämtliche Riechstoffe des Bestandteils (B), bevorzugt in einer für den, mehrere oder sämtliche Riechstoffe des Bestandteils (B) (i) fixierenden Menge, enthalten ist.

Unter einer fixierenden Menge des Bestandteils (A) ist eine Menge an Bestandteil (A) zu verstehen, die ausreicht, eine Fixierung eines, mehrerer oder sämtlicher Riechstoffe des Bestandteils (B) zu bewirken.

Vorzugsweise liegt das Massenverhältnis des Bestandteils (A) zur Gesamtmasse des Bestandteils (B) im Bereich von 1 : 20 bis 200 : 1, bevorzugt im Bereich von 1 : 12 bis 100 : 1, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1.

Bei Einstellung der (als bevorzugt) angegebenen Massenverhältnisse der Bestandteile (A) und (B) in einer erfindungsgemäßen Zusammensetzung bzw. einem erfindungsgemäßen kosmetischen (topischen) Produkt wird der fixierende Effekt des Bestandteils (A) auf Bestandteil (B) bewirkt.

Die erfindungsgemäßen Zusammensetzungen zeigen überraschenderweise eine Fixierung des, der oder sämtlicher Riechstoffe des Bestandteils (B) durch den Bestandteil (A), insbesondere bei Einstellung der als bevorzugt bezeichneten Massenverhältnissen. Durch eine fixierend wirksame Menge des Bestandteils (A), der als Fixateur für einen, mehrere oder sämtliche Riechstoffe des Bestandteils (B) wirkt, wird eine verbesserte und verlängerte Haftung (Haftfestigkeit) des Bestandteils (B) erreicht, insbesondere auf (menschlicher) Haut und/oder (menschlichem) Haar. Dies gilt ebenfalls für erfindungsgemäße kosmetische (topische) Produkte, die vorzugsweise eine sensorisch (geruchlich) wirksame Menge einer erfindungsgemäßen Zusammensetzung enthalten.

EP 2 014 749 betrifft Basisöle als Bestandteil von Kühlmitteln für Kohlendioxid betriebene Kältemaschinen. Die dort verwendeten Basisöle betreffen primär Ester von polyhydrischen Alkoholen und C14-C22 verzweigtkettigen Carbonsäuren in Mischung mit anderen Estern, unter anderem werden zwei spezielle Basisöle mit Minderanteilen von Neopentylglycoldiisononanoat beschrieben (Base oil 4und Base oil 26). Diese Basisöle enthalten keine Riechstoffe.

In bevorzugten Ausführungsformen ist eine erfindungsgemäße Zusammensetzung bzw. ein erfindungsgemäßes kosmetisches (topisches) Produkt frei von Mischungen von Neopentylglycol-di-n-dodecanat und Neopentylglycoldiisononanoat im Gewichtsverhältnis 1,4 : 15 und/oder von Neopentylglycol-di-n-tetradecanat und Neopentylglycoldiisononanoat im Gewichtsverhältnis 2 : 15 und/oder von Neopentylglycol-di-n-pentadecanat und Neopentylglycoldiisononanoat im Gewichtsverhältnis 0,7 : 15. In weiteren bevorzugten Ausführungsformen ist eine erfindungsgemäße Zusammensetzung bzw. ein erfindungsgemäßes kosmetisches (topisches) Produkt frei von Neopentylglycol-di-n-dodecanat und/oder Neopentylglycol-di-n-tetradecanat und/oder Neopentylglycol-din-pentadecanat. In weiteren bevorzugten Ausführungsformen ist eine erfindungsgemäße Zusammensetzung bzw. ein erfindungsgemäßes kosmetisches (topisches) Produkt kein Basisöl bzw. kein Kühlmittelöl wie in EP 2 014 749 beschrieben. In einer weiteren bevorzugten Ausführungsform ist eine erfindungsgemäße Zusammensetzung bzw. ein erfindungsgemäßes kosmetisches (topisches) Produkt frei von C1-C3-Fluorkohlenwasserstoffen und/oder Estern von polyhydrischen Alkoholen und C14-C22 verzweigtkettigen Carbonsäuren.

WO 94/13764 offenbart Mischungen von Neopentylglycoldiisononanoat und 1,1,1-Trifluorethan als Kühlmittel. In bevorzugten Ausführungsformen ist eine erfindungsgemäße Zusammensetzung bzw. ein erfindungsgemäßes kosmetisches (topisches) Produkt frei von 1,1,1-Trifluorethan.

US 5,202,044 und US 5,395,444 beschreiben Kühlmittel, welche auf Difluormethan basieren. Die dort beschriebenen Kühlmittel enthalten bestimmte Ester, wobei auch Neopentylglycoldiisononanoat genannt ist. Ferner können die dort beschriebenen Kühlmittel 0,001 bis 2 Gew.-% an Metalldeaktivatoren enthalten, bezogen auf die Menge an eingesetztem Ester. In der Liste der Metalldeaktivatoren sind unter anderem Substanzen genannt, die einen Geruch aufweisen, hier sind neben Ethylendiamin und Diethylmalonat insbesondere Acetylaceton und Acetessigester (Ethyl-3-oxobutanoat) zu nennen. Explizit offenbart ist eine Mischung von Neopentylglycoldiisononanoat und riechenden Metalldeaktivatoren in US 5,202,044 und US 5,395,444 nicht. In bevorzugten Ausführungsformen ist eine erfindungsgemäße Zusammensetzung bzw. ein erfindungsgemäßes kosmetisches (topisches) Produkt frei von Difluormethan. In weiteren bevorzugten Ausführungsformen ist eine erfindungsgemäße Zusammensetzung bzw. ein erfindungsgemäßes kosmetisches (topisches) Produkt entweder frei von Ethylendiamin, Diethylmalonat, Acetylaceton (welche keine Parfümerie-Riechstoffe gemäß untenstehender Definition sind) und Acetessigester (welches ein Parfümerie-Riechstoff gemäß untenstehender Definition ist) oder die erfindungsgemäße Zusammensetzung bzw. das erfindungsgemäße kosmetische Produkt enthält einen (weiteren), bevorzugt zwei, drei, vier, fünf oder mehr (weitere), Parfümerie-Riechstoffe (wie unten definiert).

In einer weiteren bevorzugten Ausführungsform enthält eine erfindungsgemäße Zusammensetzung bzw. ein erfindungsgemäßes kosmetisches Produkt keine Fluorchlorkohlenwasserstoffe, insbesondere keine C1-C2-Fluorchlorkohlenwasserstoffe, und/oder keine C1-C3-Fluorkohlenwasserstoffe, insbesondere nicht solche, die in den obigen Dokumenten (explizit) beschrieben sind. Solche Fluorchlorkohlenwasserstoffe und Fluorkohlenwasserstoffe sind geruchlos und insoweit auch nicht als Riechstoffe anzusehen, insbesondere nicht als Parfümerie-Riechstoffe gemäß untenstehender Definition.

In Kongop Hwahak 2001, 12, 334-339 wurden die Hydrolysegeschwindigkeiten verschiedener Ester, unter anderem von Neopentylglycoldiisononanoat, untersucht. Als Hydrolysekatalysator wurde dort p-Toluolsulfonsäure verwendet.

In DE 1912486 ist die Veresterung von Neopentylglycol mit einer Mischung von n-Nonansäure (Pelargonsäure) und Isononansäure (3,5,5-Trimethylhexansäure) zu 2,2-Dimethylpropan-1,3-diol-3,5,5-hexanat-pelargonat in Gegenwart von konzentrierter Schwefelsäure in Cumol als wasserschleppendem Mittel beschrieben. Unbekannt ist dabei, ob in der Reaktionsmischung auch Neopentylglycoldiisononanoat entstanden ist. DE 1912486 führt zu Neopentylglycoldiisononanoat lediglich aus, dass es schlechtere Schmierstoff-Eigenschaften aufweist als 2,2-Dimethylpropan-1,3-diol-3,5,5-hexanat-pelargonat. In bevorzugten Ausführungsformen ist eine erfindungsgemäße Zusammensetzung bzw. ein erfindungsgemäßes kosmetisches (topisches) Produkt keine (Reaktions)Mischung wie in DE 1912486 beschrieben. Vorsorglich ist in weiteren bevorzugten Ausführungsformen eine erfindungsgemäße Zusammensetzung bzw. ein erfindungsgemäßes kosmetisches (topisches) Produkt frei von 2,2-Dimethylpropan-1,3-diol-3,5,5-hexanat-pelargonat, Cumol und/oder konzentrierter Schwefelsäure.

JP 52131513 offenbart die Verwendung von Neopentylglycoldiisononanoat in Kosmetika für Haut und Haar. Diese Kosmetika können Parfüm enthalten, es werden allerdings keine Mengenangaben zu der eingesetzten Menge an Parfüm gemacht. Es werden dort ferner keine einzelnen Riechstoffe und keine geruchlichen Effekte beschrieben. Ferner offenbart JP 52131513 die Herstellung von Neopentylglycoldiisononanoat mittels azeotroper Veresterung von Neopentylglycol und Isononansäure in Xylol in Gegenwart von p-Toluolsulfonsäure.

JP 2008-162965 beschreibt reinigende kosmetische Cremes, welche allgemein 50 bis 95 Gew.-% an Neopentylglycoldiisononanoat enthalten. Ferner ist in obigem Dokument ausgeführt, dass die dort beschriebenen reinigenden kosmetischen Cremes übliche Additive enthalten können, wobei unter anderem Parfüm als Additiv genannt ist. Ausführungen zu Parfüm fehlen dort, insbesondere werden keine einzelnen Riechstoffe angegeben. Ferner werden dort keine geruchlichen Effekte beschrieben und keine Mengenangaben zu dem dort optionalen Additiv Parfüm gemacht. Ferner beschreibt JP 2008-162965 die Herstellung von Neopentylglycoldiisononanoat mittels azeotroper Veresterung von Neopentylglycol und Isononansäure in Toluol in Gegenwart von p-Toluolsulfonsäure.

Die in den obigen Dokumenten beschriebenen Veresterungsverfahren von Neopentylglycol und Isononansäure zur Herstellung von Neopentylglycoldiisononanoat nutzen aromatische Lösungsmittel bei der azeotropen Veresterung als Wasserschlepper.

Toluol, Xylole und Cumol sind keine Parfümerie-Riechstoffe gemäß untenstehender Definition. Insbesondere die in den obigen Dokumenten jeweils beschriebenen Reaktionsmischungen sind nicht Gegenstand der vorliegenden Erfindung. Dies gilt umso mehr, als diese Lösungsmittel teils stark gesundheitsgefährdend und somit nicht für den Einsatz auf dem Gebiet der vorliegenden Erfindung geeignet sind, insbesondere nicht für den Einsatz in kosmetischen (topischen) Produkten. In bevorzugten Ausführungsformen sind daher erfindungsgemäße Zusammensetzungen und kosmetische (topische) Produkte frei von Toluol, Xylolen und Cumol, bevorzugt frei von aromatischen Kohlenwasserstoffen mit einer Molmasse von kleiner als 125 g/mol.

Alternativ und vorzugsweise zusätzlich sind erfindungsgemäße Zusammensetzungen und kosmetische (topische) Produkte frei von p-Toluolsulfonsäure und frei von (konzentrierter) Schwefelsäure (H₂SO₄).

Alternativ und vorzugsweise zusätzlich sind erfindungsgemäße Zusammensetzungen und kosmetische (topische) Produkte frei von Isononansäure und/oder frei von Pelargonsäure, welche keine Parfümerie-Riechstoffe gemäß untenstehender Definition sind.

### Bestandteil (A)

Neopentylglycoldiisononanoat [CAS Nummer 27841-07-2; 2,2-Dimethyl-1,3-propandioldiisononanoat, Diester des 2,2-Dimethyl-1,3-propandiols und der 3,5,5-Trimethylhexansäure (Isononansäure); 2,2-Dimethyl-1,3-propandiol-di-3,5,5-trimethylhexanat] weist die nachfolgende Strukturformel (I) auf:

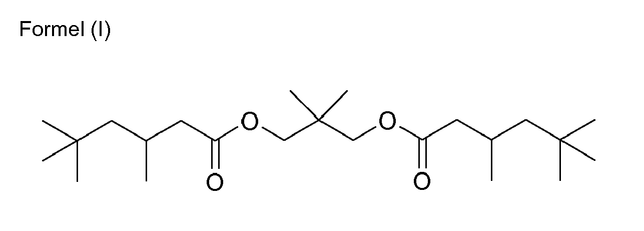

Neopentylglycoldiisononanoat kann beispielsweise mittels dem Fachmann bekannten üblichen chemischen oder biotechnologischen Veresterungsverfahren durch Umsetzung von Neopentylglycol und 3,3,5-Trimethylhexansäure hergestellt werden. Ferner ist die Verbindung der Formel (I) kommerziell erhältlich.

Bestandteil (A) einer erfindungsgemäßen Zusammensetzung eignet sich hervorragend für die gestellte Aufgabe, da Neopentylglycoldiisononanoat keinen oder allenfalls sehr geringen Eigengeruch aufweist. Bestandteil (A) ist selbstverständlich nicht Teil des Bestandteils (B).

Ferner vermittelt Neopentylglycoldiisononanoat ein angenehmes Hautgefühl, wodurch sich erfindungsgemäße Zusammensetzungen hervorragend für die Applikation auf (menschliche) Haut eignen sowie für die Einarbeitung und als Teil von kosmetischen (topischen) Produkten.

Darüberhinaus bewirkt Neopentylglycoldiisononanoat aufgrund seines guten Spreitvermögens auf der (menschlichen) Haut, dass der oder die Riechstoffe des Bestandteils (B) einer erfindungsgemäßen Zusammensetzungen oder eines erfindungsgemäßen kosmetischen (topischen) Produktes gleichmäßig auf der (menschlichen) Haut verteilt werden. In ähnlicher Weise werden ferner in solchen erfindungsgemäßen Zusammensetzungen oder Produkten gegebenenfalls vorhandene (vorzugsweise lipophile) (Wirk)Stoffe gleichmäßiger auf der Haut verteilt.

### Bestandteil (B)

Beispiele für Riechstoffe des Bestandteils (B), ebenso der (bevorzugten) Bestandteile (B*), (B) (i) und (B) (ii), sind dem Fachmann bekannt und beispielsweise zu finden in S. Arctander, Perfume and Flavor Materials, Vol. I und II, Montclair, N. J., 1969, Selbstverlag oder H. Surburg, J. Panten, "Common Fragrance and Flavor Materials", 5th. Ed., Wiley-VCH, Weinheim 2006.

Vorzugsweise handelt es sich bei Bestandteil (B) um eine Riechstoffmischung, bevorzugt um eine Riechstoffmischung umfassend 3, 4, 5, 6, 7, 8, 9, 10 oder mehr Riechstoffe, weiter bevorzugt um Parfümerie-Riechstoffe, insbesondere um ein Parfümöl.

Bevorzugte Riechstoffe des Bestandteils (B) sind Parfümerie-Riechstoffe, welche wiederum bevorzugt gewählt sind aus der Gruppe (B*) bestehend aus der Gruppe der als Riechstoffe offenbarten Einzelverbindungen gemäß
- S. Arctander, Perfume and Flavor Materials, Vol. I und II, Montclair, N. J., 1969, Selbstverlag ("Arctander"),
- H. Surburg, J. Panten, "Common Fragrance and Flavor Materials", 5th. Ed., Wiley-VCH, Weinheim 2006 ("Surburg"),
und
- den im Rahmen des vorliegenden Textes als Riechstoffe bezeichneten Einzelverbindungen.

Sollte im Einzelfall eine mangelnde Übereinstimmung oder ein Widerspruch zwischen diesen drei Stellen bestehen, hat der vorliegende Text Vorrang vor "Arctander" und "Surburg". Sollte im Einzelfall eine mangelnde Übereinstimmung oder ein Widerspruch zwischen "Surburg" und "Arctander" bestehen, hat "Surburg" Vorrang vor "Arctander".

In einer bevorzugten Ausgestaltung betrifft die vorliegende Erfindung eine Zusammensetzung umfassend (oder bestehend aus):
(A) Neopentylglycoldiisononanoat,
   und
(B) einem oder mehreren Riechstoffen der Gruppe (B*) wie oben definiert, vorzugsweise einer Riechstoffmischung umfassend 2, 3, 4, 5, 6, 7, 8, 9, 10 oder mehr Riechstoffe der Gruppe (B*), wobei (B*)
(B) (i) einen, 2, 3, 4, 5 oder mehr Riechstoffe mit einem Molgewicht im Bereich von 100 g/mol bis 175 g/mol (Kopfnote, Topnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
und
wobei Bestandteil (A) vorzugsweise in einer für den, mehrere oder sämtliche Riechstoffe des Bestandteils (B*), bevorzugt in einer für den, mehrere oder sämtliche Riechstoffe des Bestandteils (B) (i) fixierenden Menge, enthalten ist.

Vorzugsweise liegt das Massenverhältnis des Bestandteils (A) zur Gesamtmasse des Bestandteils (B*) im Bereich von 1 : 20 bis 200 : 1, bevorzugt im Bereich von 1 : 12 bis 100 : 1, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1. Weiter bevorzugte erfindungsgemäße Zusammensetzungen sind solche, bei denen Bestandteil (B), vorzugsweise Bestandteil (B*),
(B) (i) einen oder mehrere Riechstoffe mit einem Molgewicht im Bereich von 100 g/mol bis 175 g/mol (Topnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
   und
(B) (ii) einen, 2, 3, 4, 5 oder mehr Riechstoffe mit einem Molgewicht von größer oder gleich 190 g/mol (Fondnote) enthält, vorzugsweise mit einem Molgewicht im Bereich von 190 g/mol bis 300 g/mol, bevorzugt mit einem Molgewicht im Bereich von 195 g/mol bis 290 g/mol, und besonders bevorzugt im Bereich 200 bis 275 g/mol.

In einer bevorzugten Ausführungsform enthält eine erfindungsgemäße Zusammensetzung zumindest 2, 3, 4, 5 oder mehr Riechstoffe des Bestandteils (B) (i), und/oder zumindest 2, 3, 4, 5 oder mehr Riechstoffe des Bestandteils (B) (ii).

Erfindungsgemäße Zusammensetzungen, die einen oder mehrere (als bevorzugt bezeichnete) Riechstoffe des Bestandteils (B) (i), insbesondere in den nachfolgend angegebenen Mengenanteilen, und einen oder mehrere Riechstoffe des Bestandteils (B) (ii) enthalten, zeigen darüberhinaus eine geruchliche Harmonisierung und Abrundung, wodurch ein eleganterer und somit geruchlich wertvollerer Geruchseindruck entsteht. Dies gilt insbesondere für die Moschusriechstoffe und weiteren nachfolgend als bevorzugt bezeichneten Fondnoten des Bestandteils (B) (ii), insbesondere in den nachfolgend angegebenen Mengenanteilen.

Die (bevorzugten) Riechstoffe der Bestandteile (B), (B*), (B) (i) sowie (B) (ii) können dabei, sofern zutreffend, in Form ihrer jeweiligen Diastereomere, Enantiomere und/oder Doppelbindungsisomere vorliegen. So können diese als (E)/(Z)-Isomere, als beliebiges Gemisch der Enantiomeren, insbesondere als Racemat, oder auch als beliebiges Gemisch der entsprechenden Diastereomeren, vorliegen.

### Bestandteil (B) (i)

Die Riechstoffe des Bestandteils (B) (i) sind als Kopfnoten (Topnoten) einer erfindungsgemäßen Zusammensetzung bzw. eines erfindungsgemäßen kosmetischen Produktes anzusehen. Die Kopfnote bestimmt den Anfangsgeruch (Angeruch) einer Riechstoffmischung bzw. eines Parfümöls.

Das Molgewicht der Riechstoffe des Bestandteils (B) (i) liegt im Bereich von 100 g/mol bis 175 g/mol (Kopfnote), vorzugsweise im Bereich von 110 g/mol bis 165 g/mol, bevorzugt im Bereich von 115 g/mol bis 160 g/mol, und besonders bevorzugt im Bereich von 120 g/mol bis 155 g/mol.

Es hat sich ferner in eigenen Untersuchungen gezeigt, dass solche Riechstoffe des Bestandteils (B) (i) besonders gut durch Bestandteil (A) fixiert werden, die ausgewählt sind aus der Gruppe bestehend aus:
- der Gruppe der (vorzugsweise nicht-aromatischen) Alkohole, dabei wiederum bevorzugt sind Terpenalkohole und aliphatische Alkohole,
- der Gruppe der (vorzugsweise nicht-aromatischen) Ketone,
- der Gruppe der (vorzugsweise nicht-aromatischen) Aldehyde,
- der Gruppe der (vorzugsweise nicht-aromatischen) Nitrile, und
- der Gruppe der (vorzugsweise nicht-aromatischen) Ester.

Die im folgenden als "nicht-aromatisch" bezeichneten Riechstoffe im Rahmen der vorliegenden Erfindung sind solche, die im strukturellen Sinne keinen aromatischen Ring, d.h. keine Phenylgruppe, enthalten.

Vorzugsweise umfasst Bestandteil (B) (i) einer erfindungsgemäßen Zusammensetzung (insbesondere in einer der als bevorzugt gekennzeichneten Ausgestaltungen) einen oder mehrere Riechstoffe aus der Gruppe bestehend aus: (B) (i) n-Heptanol, trans-9-Decen-1-ol, Campher, alpha-Pinen, beta-Pinen, gamma-Terpinen, alpha-Phellandren, alpha-Terpineol, Borneol, Limonen, 6-Methyl-5-hepten-2-on, n-Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Benzaldehyd, Linalool, Tetrahydrolinalool, Tetrahydrogeraniol, Citral, Neral, Geranial, Benzylalkohol, Benzylacetat, Methylcinnamat, p-Anisaldehyd, Menthol, Isoamylacetat, Isoamylbutyrat, cis-3-Hexenylacetat, trans-3-Hexenylacetat, Hexylacetat, Butylbutyrat, Citronellol, Nerol, Geraniol, 2-Phenylethylalkohol, Methylbenzoat, Methylanthranilat, Styrallylacetat, 2,4-Dimethyl-3-cyclohexencarboxaldehyd (Vertocitral), Acetophenon, p-Methylacetophenon, Zimtalkohol, Zimtaldehyd, 3-Phenylpropanol (Dihydrozimtalkohol), 2-Methyl-1-phenyl-2-propanol (alpha,alpha-Dimethylphenethylcarbinol), Anethol, Anisalkohol, Heliotropin, Acetanisol (p-Methoxyacetophenon) und Agrunitril (3,7-Dimethyl-6-octen-1-nitril).

Bevorzugt sind folgende Riechstoffe des Bestandteils (B) (i), welche "nicht-aromatisch" im strukturellen Sinne sind, da bei diesen eine höhere Retention erzielt wurde: n-Heptanol, trans-9-Decen-1-ol, Campher, alpha-Pinen, beta-Pinen, gamma-Terpinen, alpha-Phellandren, alpha-Terpineol, Borneol, Limonen, 6-Methyl-5-hepten-2-on, n-Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Linalool, Tetrahydrolinalool, Tetrahydrogeraniol, Citral, Neral, Geranial, Menthol, Isoamylacetat, Isoamylbutyrat, cis-3-Hexenylacetat, trans-3-Hexenylacetat, Hexylacetat, Butylbutyrat, Citronellol, Nerol, Geraniol, 2,4-Dimethyl-3-cyclohexencarboxaldehyd (Vertocitral) und Agrunitril (3,7-Dimethyl-6-octen-1-nitril).

Besonders bevorzugt sind folgende Riechstoffe des Bestandteils (B) (i), da bei diesen eine noch höhere Retention erzielt wurde: n-Heptanol, Campher, alpha-Pinen, beta-Pinen, alpha-Terpineol, Limonen, 6-Methyl-5-hepten-2-on, n-Octanal, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Linalool, Tetrahydrolinalool, Tetrahydrogeraniol, Citral, Neral, Geranial, Menthol, Isoamylacetat, Isoamylbutyrat, cis-3-Hexenylacetat, Hexylacetat, Citronellol, Nerol, Geraniol, 2,4-Dimethyl-3-cyclohexencarboxaldehyd (Vertocitral) und Agrunitril (3,7-Dimethyl-6-octen-1-nitril).

Am meisten bevorzugt sind folgende Riechstoffe des Bestandteils (B) (i), da bei diesen die höchste Retention erzielt wurde:

n-Heptanol, Campher, Limonen, Dihydromyrcenol (2,6-Dimethyl-7-octen-2-ol), Linalool, Tetrahydrolinalool, Citronellol, Geraniol und Agrunitril (3,7-Dimethyl-6-octen-1-nitril).

Bei Einsatz der als (besonders) bevorzugt bezeichneten Riechstoffe der Gruppe (B) (i) sind die genannten Effekte besonders ausgeprägt.

Die Gesamtmenge des oder der (als bevorzugt bezeichneten) Riechstoffe des Bestandteils (B) (i) (Kopfnote) einer erfindungsgemäß bevorzugten Zusammensetzung bzw. eines erfindungsgemäß bevorzugten kosmetischen Produktes beträgt 5 bis 80 Gew.-%, besonders bevorzugt 10 bis 70 Gew.-%, jeweils bezogen auf das Gesamtgewicht des Bestandteils (B), vorzugsweise jeweils bezogen auf das Gesamtgewicht des Bestandteils (B*).

### Bestandteil (B) (ii)

Die Riechstoffe des optionalen Bestandteils (B) (ii) sind als Fondnoten einer erfindungsgemäßen Zusammensetzung bzw. eines erfindungsgemäßen kosmetischen Produktes anzusehen. Die Fondnote bestimmt den Nachgeruch einer Riechstoffmischung bzw. eines Parfümöls.

Das Molgewicht der Riechstoffe des Bestandteils (B) (ii) ist größer oder gleich 190 g/mol und liegt bevorzugt im Bereich von 190 g/mol bis 300 g/mol, weiter bevorzugt im Bereich von 195 g/mol bis 290 g/mol, und am meisten bevorzugt im Bereich von 200 bis 275 g/mol.

Mit solchen Riechstoffen des Bestandteils (B) (ii) können die beschriebenen Effekte der vorliegenden Erfindung am ausgeprägtesten und deutlichsten erreicht werden

Bevorzugte erfindungsgemäße Zusammensetzungen sind solche, in denen Bestandteil (B) (ii) einen, zwei, drei oder mehr Riechstoffe enthält, gewählt aus der Gruppe bestehend aus:
- Moschusriechstoffen,
   alpha-n-Amylzimtaldehyd (MW = 202,30), alpha-iso-Amylzimtaldehyd (MW = 202,30), alpha-n-Hexylzimtaldehyd (MW = 216,32), alpha-iso-Hexylzimtaldehyd (MW = 216,32), Benzylsalicylat (MW = 228,25), cis-3-Hexenylsalicylat (MW = 220,27), Isoamylsalicylat (MW = 208,26), Hexylsalicylat (MW = 222,28), 2-Methyl-3-(4-tert-butylphenyl)propanal (MW = 204,31; Lilial®), 2-Methyl-3-(4-isopropylphenyl)propanal (MW = 190,28, Cyclamenaldehyd), 2,3,8,8-Tetramethyl-1,2,3,4,5,6,7,8-octahydro-2-naphthalenylmethylketon (MW = 234,38, Iso E Super®), 1-[1,2,3,4,6,7,8,8a-octahydro-1,2,8,8-tetramethylnaphtalen-2-yl]ethanon (MW = 234,38, Iso E Super®), Methyldihydrojasmonat (MW = 226,32, Hedione®), Linalylacetat (MW = 196.29), Ethyllinalylacetat (MW = 210,31), Nerolidol (MW = 222,37), Farnesol (MW = 222,37), Cedrylmethylether (MW = 236,40, Cedramber), Cedrylmethylketon (MW = 246,39), Cedrylacetat (MW = 264,41), (4aR,5R,7aS,9R)-Octahydro-2,2,5,8,8,9a-hexamethyl-4H-4a,9-Methanoazuleno(5,6-d) 1,3-dioxol) (MW = 278,44, Ambrocenide®), Hexahydro-1',1',5',5'-tetramethyl-spiro[1,3-dioxolan,2,8' (5'H)-[2H-2,4a]-methanonaphthalin (MW = 264,41 Ethylendioxi-3H-isolongifolan, Ysamber® K), 2-Methyl-4-(2,2,3-trimethyl-3-cyclopenten-1-yl)butanol (MW = 196,34, Brahmanol), 5-(2,2,3-Trimethyl-3-cyclopenten-1-yl)-3-methylpentan-2-ol (MW = 210,36, Sandalore®), 2-Ethyl-4-(2,2,3-trimethyl-3-cyclopenten-1-yl)-2-buten-1-ol (MW = 208,35, Sandranol®), 3-Methyl-5-(2,2,3-trimethyl-3-cyclopenten-1-yl)-4-penten-2-ol (MW = 208,35, Ebanol®), 3,3-Dimethyl-5-(2,2,3-trimethyl-3-cyclopenten-1-yl)-4-penten-2-ol (MW = 222,37, Polysantol®), 3-Isocamphylcyclohexanol (MW = 236,40, Sandel 80®),1-(2,2,6-Trimethylcyclohexyl)hexan-3-ol (MW = 226,41, Timberol®), Cyclododecylmethylether (MW = 198,35, Palisandin), (Ethoxymethoxy)cyclododecan (MW = 242,41, Boisambrene forte®), 1-Methyl-4-(4-methyl-3-penten-1-yl)-3-cyclohexencarboxaldehyd (MW = 206,33, Precyclemone B®), 4-(4-Hydroxy-4-methylpentyl)-3-cyclohexencarboxaldehyd (MW = 210,32, Lyral®), 2-Methyl-4-(2,2,6-trimethyl-1-cyclohexen-1-yl)-2-butenal (MW = 206,33, Boronal), Decahydro-beta-naphthylacetat (MW = 196,29), Allyl-3-cyclohexylpropionat (MW = 196,29), Allylcyclohexyloxyacetat (MW = 198,26, Isoananat®), Citraldiethylacetal (MW = 226,36), Benzylbenzoat (MW = 212,25), Benzylcinnamat (MW = 238,29), 3a,6,6,9a-Tetramethyldodecahydronaphtho[2,1-*b*]furan (MW = 236,40, Ambroxid®), alpha-Iron (MW = 206,33), beta-Iron (MW = 206,33), alpha-n-Methylionon (MW = 206,33), beta-n-Methylionon (MW = 206,33), alpha-Isomethylionon (MW = 206,33), beta-Isomethylionon (MW = 206,33) und Allylionon (MW 232,35). 2,3,8,8-Tetramethyl-1,2,3,4,5,6,7,8-octahydro-2-naphthalenylmethylketon (MW = 234,38), 1-[1,2,3,4,6,7,8,8a-octahydro-1,2,8,8-tetramethylnaphtalen-2-yl]ethanon (MW = 234,38), Isobornylacetat (MW = 196,29), alpha-Ionon (MW = 192,30), beta-Ionon (MW = 192,30), gamma-Ionon (MW = 192,30), alpha-Damascon (MW = 192,30), beta-Damascon (MW = 192,30), delta-Damascon (MW = 192,30), 1-(2,4,4-Trimethyl-2-cyclohexen-1-yl)-2-buten-1-on (MW = 192,30, Isodamascon), Cedrol (MW = 222,40), gamma-Dodecalacton (MW = 198,30), 2-Methyl-3-(3,4-methylendioxyphenyl)propanal (MW = 192,22, Helional) und Methyldihydrojasmonat (MW = 226,31).

In Klammern sind dabei die Molgewichte (MW) und ggf. übliche Marken- bzw. Produktnamen angegeben.

Der oder die Moschusriechstoffe des Bestandteils (B) (ii) sind Riechstoffe, die einen Moschusgeruch aufweisen. Solche Riechstoffe sind dem Fachmann bekannt, da "Moschus" (musk) eine sehr wichtige Geruchsrichtung in der Parfümerie darstellt. Ferner sind Moschusriechstoffe, unter anderem die unten bevorzugt genannten, beschrieben in H. Surburg, J. Panten, "Common Fragrance and Flavor Materials", 5th. Edition, Wiley-VCH, Weinheim 2006.

Der oder die Moschusriechstoffe, die Teil des Bestandteils (B) (ii) sind, sind dabei vorzugsweise gewählt aus der Gruppe der macrocyclischen Moschusriechstoffe, der polycyclischen Moschusriechstoffe und/oder der alicyclischen Moschusriechstoffe.

Erfindungsgemäß bevorzugt sind Bestandteile (B), insbesondere Parfümerie-Riechstoffmischungen, enthaltend zwei, drei oder mehrere verschiedene Moschusriechstoffe als Bestandteil (B) (ii).

Bevorzugt werden der oder die Moschusriechstoffe des Bestandteils (B) (ii) im Rahmen der vorliegenden Erfindung gewählt aus nachfolgender Tabelle 1:

**Tabelle 1:**

| TYP | Produkt / Markenname | Name / CAS-Name |
|---|---|---|
| MACRO | EXALTENON | 4-Cyclopentadecen-1-one (4Z)- ; 4-Cyclopentadecen-1-on |
| MACRO | CIVETON | 9-Cycloheptadecen-1-on, (9Z)- |
| MACRO | CYCLOHEXADECANOLID, DIHYDROAMBRETTOLID | Oxacycloheptadecan-2-on, ω-Hexadecanolid |
| MACRO | ETHYLENDODECANDIOAT | 1,4-Dioxacyclohexadecane-5,16-dion |
| MACRO | GLOBALIDE® | Oxacyclohexadecen-2-on; 15-Pentadec-(11/12)-enolid |
| MACRO | ETHYLENBRASSYLAT | 1,4-Dioxacycloheptadecane-5,17-dion |
| MACRO | MUSCON | 3-Methy-cyclopentadecanon |
| MACRO | AMBRETTOLID | Oxacycloheptadec-10-en-2-on |
| MACRO | MUSCENON | 3-Methyl-cyclopentadecenon |
| MACRO | VELVIONE®, AMBRETONE | 5-Cyclohexadecen-1-on |
| MACRO | AURELIONE® | 7/8-Cyclohexadecen-1-on |
| MACRO | GLOBANONE® | 8-Cyclohexadecen-1-on |
| MACRO | ISOMUSCONE® | Cyclohexadecanon |
| MACRO | EXALTOLID, MACROLIDE® | Oxacyclohexadecan-2-on |
| MACRO | COSMONE® | 3-Methyl-(5E/Z)-cyclotetradecen-1-on |
| POLY | TRASEOLIDE® | 1-[2,3-Dihydro-1,1,2,6-tetramethyl-3-(1-methylethyl)-1H-inden-5- yl]-ethanon |
| POLY | PHANTOLIDE® | 1-(2,3-Dihydro-1,1,2,3,3,6-hexamethyl-1H-inden-5-yl)-ethanon |
| POLY | TONALIDE® | 1-(5,6,7,8-Tetrahydro-3,5,5,6,8,8-hexamethyl-2-naphthalenyl)-ethanon |
| POLY | CRYSOLIDE | 1-[6-(1,1-Dimethylethyl)-2,3-dihydro-1,1-dimethyl-1H-inden-4-yl]-ethanon |
| POLY | CHROMANOLIDE® | Tetradecansäure, 1-methylethyl ester; Cyclopenta[g]-2-benzopyran, 1,3,4,6,7,8-hexahydro-4,6,6,7,8,8-hexamethyl- |
| POLY | GALAXOLIDE® | Cyclopenta[g]-2-benzopyran, 1,3,4,6,7,8-hexahydro-4,6,6,7,8,8-hexamethyl- |
| ALICYC | HELVETOLIDE® | 1-Propanol, 2-[1-(3,3-dimethylcyclohexyl)ethoxy]-2-methyl-, 1-propanoat |

| | | |
|---|---|---|
| MACRO = macrocyclische Moschusriechstoffe POLY = polycyclische Mochusriechstoffe ALICYC = alicyclischer Mochusriechstoff | | |

Weiter bevorzugt als Teil des Bestandteils (B) (ii) sind polycyclische und/oder macrocyclische Moschusriechstoffe, wobei sich insbesondere macrocyclische Moschusriechstoffe als besonders vorteilhaft im Sinne der Erfindung gezeigt haben, welche wiederum vorzugsweise gewählt sind aus der Gruppe bestehend aus macrocyclischen C₁₄-C₁₈-Ketonen und macrocyclischen C₁₄-C₁₈-Lactonen, wobei das Keton bzw. Lacton eine Ringgröße von 15 bis 17 Ringatomen und kein, ein oder zwei Sauerstoffatome im Ring aufweist.

Am meisten bevorzugt sind 3-Methylcyclopentadecenon (Muscenon), 15-Pentadec-(11/12)-enolid (Globalide®), Ethylenbrassylat, Oxacyclohexadecan-2-on (Macrolide®), Cyclohexadecanon (Isomuscone®), 8-Cyclohexadecanon (Globanone®), (7/8)-Cyclohexadecanon (Aurelione®) und deren Mischungen.

In einer bevorzugten Ausführungsform wird Bestandteil (B) (ii) gewählt aus der Gruppe bestehend aus 15-Pentadec-(11/12)-enolid (Globalide)®, Ethylenbrassylat und Oxacyclohexadecan-2-on (Macrolide®) und deren Mischungen.

Die Gesamtmenge des oder der (als bevorzugt bezeichneten) Riechstoffe des Bestandteils (B) (ii) (Fondnote) einer erfindungsgemäß bevorzugten Zusammensetzung bzw. eines erfindungsgemäß bevorzugten kosmetischen Produktes beträgt 5 bis 80 Gew.-%, besonders bevorzugt 10 bis 70 Gew.-%, jeweils bezogen auf das Gesamtgewicht des Bestandteils (B), vorzugsweise jeweils bezogen auf das Gesamtgewicht des Bestandteils (B*).

In einer erfindungsgemäßen Zusammensetzung oder einem erfindungsgemäßen kosmetischen Produkt (jeweils insbesondere in einer der als bevorzugt angegebenen Ausgestaltungen) werden vorzugsweise zusätzlich einer oder mehrere Effekte bewirkt, die ausgewählt sind aus der Gruppe von Effekten bestehend aus:
- Reduzierung der Verdampfung von Riechstoffen, insbesondere unmittelbar nach der Applikation der erfindungsgemäßen Zusammensetzung und insbesondere der Kopfnote; dies gilt auch für (leichtflüchtige) Riechstoffe, die üblicherweise durch die Verdampfung von Lösungsmitteln wie Ethanol/Wasser kurz nach deren Applikation mitgerissen werden;
- zeitliche Stabilisierung des Geruchsprofils, das heißt, dass über einen längeren Zeitraum ein gleichbleibenderes Geruchsprofil erreicht wird;
- Erhöhung oder zeitliche Stabilisierung des geruchlichen Impacts (der wahrgenommenen Geruchsstärke); das heißt, dass der geruchliche Impact (die wahrgenommene Geruchsstärke) über einen langen Zeitraum nicht oder nicht nennenswert verringert wird;
- Verlängerung der Haftung der Riechstoffe des Bestandteils (B) einer erfindungsgemäßen Zusammensetzung, insbesondere der leichtflüchtigen Riechstoffe (Kopfnote), dabei wiederum vorzugsweise die als bevorzugt bezeichneten Riechstoffe des Bestandteils (B) (i), auf Haut und/oder Haar, insbesondere auf menschlicher Haut, wobei die Retention (Rückhaltung) des Bestandteils (B) einer erfindungsgemäßen Zusammensetzung oder eines erfindungsgemäßen kosmetischen Produktes höher ist im Vergleich zu einer ansonsten identischen Zusammensetzung oder einem ansonsten identischen Produkt, welche(s) keinen Bestandteil (A) enthält;
- Vermittlung eines angenehmen Hautgefühls einer (ethanolischen) Riechstoffzusammensetzung.

Die erfindungsgemäßen Zusammensetzungen sind vorzugsweise Bestandteil von kosmetischen, insbesondere topischen, kosmetischen Produkten. Bei einer erfindungsgemäßen Zusammensetzung oder einem erfindungsgemäßen kosmetischen (topischen) Produkt enthaltend eine sensorisch (geruchlich) wirksame Menge einer erfindungsgemäßen Zusammensetzung, wird eine Verlängerung der Haftung (Fixierung) der Riechstoffe des Bestandteils (B) bewirkt, bevorzugt auf Haut und/oder Haar, insbesondere auf menschlicher Haut. Dabei werden vorzugsweise ein oder mehrere weitere im Rahmen der vorliegenden Erfindung beschriebenen Effekte erzielt, insbesondere bei den als bevorzugt bezeichneten Ausgestaltungen.

Die verlängerte Haftung (Fixierung) der Riechstoffe des Bestandteils (B) wurde insbesondere bei leichtflüchtigen Riechstoffen (Kopfnote) beobachtet, dabei wiederum vorzugsweise der als bevorzugt bezeichneten Riechstoffe des Bestandteils (B) (i), auf Haut und/oder Haar, insbesondere auf menschlicher Haut.

Die Retention (Rückhaltung) des Bestandteils (B) einer erfindungsgemäßen Zusammensetzung oder eines erfindungsgemäßen kosmetischen Produktes ist vorzugsweise mindestens 40 Gew.-%, bevorzugt mindestens 50 Gew.-%, weiter bevorzugt mindestens 80 Gew.-%, besonders bevorzugt mindestens 100 Gew.-%, höher im Vergleich zu einer ansonsten identischen Zusammensetzung oder einem ansonsten identischen Produkt, welche(s) keinen Bestandteil (A) enthält, vorzugsweise ermittelt wie untenstehend in Test 1 beschrieben.

Erfindungsgemäße Zusammensetzungen und kosmetische Produkte sind demnach - kurz zusammengefasst - Zusammensetzungen / Produkte mit einer besonders guten Haftung der Kopfnote einer Riechstoffzusammensetzung, einem verlängerten und/oder verstärkten Impact der Kopfnote, wobei vorzugsweise eine Verstärkung und/der Verlängerung der Fondnote nicht oder zumindest nicht in sensorisch nennenswertem Umfang zu beobachten ist. Es findet also insgesamt eine Diskriminierung hinsichtlich der Fixierung von Kopf- und Fondnoten durch Bestandteil (A) in erfindungsgemäßen Zusammensetzungen und Produkten statt. Die Haftfestigkeit bzw. die Wahrnehmbarkeit, insbesondere der Kopfnote, von Riechstoffzusammensetzungen, dabei insbesondere auf menschlicher Haut, wird dadurch verlängert und somit eine verbesserte geruchliche "Profilstabilität" erreicht.

Eine erfindungsgemäße Zusammensetzung oder ein (kosmetisches) Produkt (insbesondere in einer der vorstehend als bevorzugt gekennzeichneten Ausgestaltungen) umfasst in einer bevorzugten Ausgestaltung als weiteren Bestandteil eine wirksame Menge
(C) eines oder mehrerer kosmetisch akzeptabler Lösungsvermittler für Bestandteil (B), vorzugsweise für Bestandteil (B*), (B) (i) und/oder (B) (ii), vorzugsweise gewählt aus der Gruppe bestehend aus
   (i) Ethanol
      und
   (ii) Dipropylenglycol, Diethylphtalat, Triethylcitrat, Isopropylmyristat und Triacetin, dabei bevorzugt ist Dipropylenglycol.

Lösungsvermittler gemäß Bestandteil (C), insbesondere die als bevorzugt angegebenen kosmetisch akzeptablen Lösungsvermittler, werden im Rahmen dieses Textes nicht als Riechstoffe aufgefasst, insbesondere nicht als Parfümerie-Riechstoffe. Bestandteil (C) ist im Rahmen der vorliegenden Erfindung nicht als Teil des Bestandteils (B) anzusehen und wird diesem folglich nicht zugerechnet.

Bestandteil (C) ist als Lösungsvermittler für Bestandteil (B) vorteilhaft, so dass eine bessere Mischbarkeit und (Ent)Mischungsstabilität von Bestandteil (B) mit Bestandteil (A) gegeben ist, wodurch eine leichtere Handhabung und bessere Weiterverarbeitung ermöglicht wird, was bei der Herstellung von erfindungsgemäßen kosmetischen Produkten von Vorteil ist.

Sofern eine erfindungsgemäße Zusammensetzung bzw. ein erfindungsgemäßes kosmetisches Produkt einen Lösungsvermittler gemäß Bestandteil (C) enthält, der gleichzeitig ein Riechstoff ist, wird dieser, insbesondere für quantitative Betrachtungen, als Bestandteil (B) angesehen und diesem zugeordnet.

Sofern eine erfindungsgemäße Zusammensetzung bzw. ein erfindungsgemäßes kosmetisches Produkt einen Lösungsvermittler gemäß Bestandteil (C) enthält, der gleichzeitig ein Diol- oder Triol gemäß Bestandteil (D) ist, wird dieser, insbesondere für quantitative Betrachtungen, als Bestandteil (D) angesehen und diesem zugeordnet.

Sofern ein erfindungsgemäßes kosmetisches Produkt Bestandteil (C) (i) Ethanol enthält, kann die Gesamtmenge des Bestandteils (C) (i) vorzugsweise bis zu 95 Gew.-% Ethanol, bevorzugt höchstens 90 Gew.-%, betragen, jeweils bezogen auf das Gesamtgewicht des erfindungsgemäßen Produktes.

Eine erfindungsgemäße Zusammensetzung bzw. ein erfindungsgemäßes kosmetisches Produkt enthält Bestandteil (C) (ii) vorzugsweise in einer Gesamtmenge von bis zu 80 Gew.-%, bevorzugt von 0,5 bis 60 Gew.-%, bevorzugt von 1 bis 50 Gew.-%, weiter bevorzugt von 5 bis 40 Gew.-%, jeweils bezogen auf das Gesamtgewicht des Bestandteils (B).

Eine erfindungsgemäße Zusammensetzung (insbesondere in einer der vorstehend als bevorzugt gekennzeichneten Ausgestaltungen) umfasst in einer alternativen bevorzugten Ausgestaltung als zusätzlichen Bestandteil
(D) ein oder mehrere Di- oder Triole mit 3 bis 12 C-Atomen, vorzugsweise gewählt aus der Gruppe bestehend aus Glycerin, 1,2-Propylenglykol, 1,2-Butylenglykol, 1,3-Butylenglykol und Alkandiolen mit 5 bis 12 C-Atomen.

Sofern eine erfindungsgemäße Zusammensetzung bzw. ein erfindungsgemäßes kosmetisches Produkt ein Diol oder Triol gemäß Bestandteil (D) enthält, welches gleichzeitig ein Riechstoff ist, wird dieses, insbesondere für quantitative Betrachtungen, als Bestandteil (D) angesehen und diesem zugeordnet. Bei Bestandteil (D), insbesondere bei den als bevorzugt angegebenen Diolen und Triolen, handelt es sich nicht um Riechstoffe, insbesondere nicht um Parfümerie-Riechstoffe.

Sofern eine erfindungsgemäße Zusammensetzung bzw. ein erfindungsgemäßes kosmetisches Produkt ein Diol- oder Triol gemäß Bestandteil (D) enthält, welches gleichzeitig einen Lösungsvermittler gemäß Bestandteil (C) ist, wird dieses, insbesondere für quantitative Betrachtungen, als Bestandteil (D) angesehen und diesem zugeordnet.

Die Alkandiole mit 5 bis 12 C-Atomen sind dabei vorzugsweise gewählt aus der Gruppe der geradkettigen 1,2-Alkandiole mit 5 bis 10 C-Atomen, insbesondere aus der Gruppe bestehend aus 1,2-Pentandiol, 1,2-Hexandiol, 1,2-Octandiol und/oder 1,2-Decandiol.

Besonders bevorzugte Di- oder Triole des Bestandteils (D) sind gewählt aus der Gruppe bestehend aus Glycerin, 1,2-Propylenglykol, 1,2-Butylenglykol, 1,3-Butylenglykol, 1,2-Pentandiol, 1,2-Hexandiol, 1,2-Octandiol und 1,2-Decandiol.

Bestandteil (D) kann eventuelle unerwünschte Trübungen reduzieren oder verhindern. Zudem kann durch Bestandteil (D) zusätzlich die Haftung einer erfindungsgemäßen Zusammensetzung auf (menschlicher) Haut und/oder (menschlichem) Haar, insbesondere auf menschlicher Haut, und/oder der geruchliche Profilverlauf einer erfindungsgemäßen Zusammensetzung weiter verbessert werden.

Eine erfindungsgemäße kosmetische Zubereitung enthält Bestandteil (D) vorzugsweise in einer Gesamtmenge von 0,2 bis 20 Gew.-%, bevorzugt von 0,5 bis 10 Gew.-%, weiter bevorzugt von 1 bis 5 Gew.-%, jeweils bezogen auf das Gesamtgewicht der kosmetischen Zubereitung.

In einer erfindungsgemäßen Zusammensetzung (insbesondere in einer der vorstehend als bevorzugt angegebenen Ausgestaltungen) liegt Bestandteil (D) vorzugsweise in einer wirksamen Menge vor, das heißt in einer Menge, bei der in der Zusammensetzung durch den Bestandteil (D) einer oder mehrere Effekte bewirkt und/oder verstärkt werden, die ausgewählt sind aus der Gruppe von Effekten bestehend aus:
- weitere Reduzierung der Verdampfung von Riechstoffen, insbesondere unmittelbar nach der Applikation der erfindungsgemäßen Zusammensetzung und insbesondere der Kopfnote;
- eine weiter verlängerte Haftung auf (menschlicher) Haut und/oder (menschlichem) Haar, insbesondere auf menschlicher Haut (damit ist auch hier menschliche Haut gemeint, bei der es sich nicht um (Mund)Schleimhaut handelt), insbesondere der Topnote;
- einen über eine längere Zeit weitgehend gleichbleibenden Geruchseindruck nach Applikation auf die (menschliche) Haut;
- einen höheren Impact nach Applikation auf die (menschliche) Haut;
- weitere zeitliche Stabilisierung des Geruchsprofils, das heißt, dass über einen längeren Zeitraum ein noch gleichbleibenderes Geruchsprofil erreicht wird;
- Erhöhung oder weitere zeitliche Stabilisierung des geruchlichen Impacts (der wahrgenommenen Geruchsstärke); abhängig von den jeweiligen eingesetzten Konzentrationen des Bestandteils (D) innerhalb der erfindungsgemäßen Zusammensetzung wird der Impact über einen mehr oder weniger langen Zeitraum erhöht;
- Verlängerung der Haftung der Zusammensetzung, insbesondere der leichtflüchtigen Riechstoffe (Kopfnote), auf Haut und/oder Haar, insbesondere auf menschlicher Haut,
- Erhöhung oder Verlängerung der Diffusivität (Raumwirkung) der Zusammensetzung, insbesondere der leichtflüchtigen Riechstoffe (Kopfnote), auf Haut und/oder Haar, insbesondere auf menschlicher Haut;
- Vermittlung eines weiter verbesserten angenehmen Hautgefühls.

Eine erfindungsgemäße Zusammensetzung hinterlässt vorzugsweise keinen Film auf Haut und/oder Haar und wird somit nicht als störend empfunden. Kurze Zeit nach dem Auftragen (das heißt nach dem Trocknen und ggf. Einziehen) ist eine erfindungsgemäße Zusammensetzung regelmäßig nicht mehr sichtbar (Ausnahme: Haarpflegemittel wie beispielsweise Haarspray, bei denen die Filmbildung gewünscht ist).

Weitere Stoffe, die Teil einer erfindungsgemäßen (kosmetischen) Zusammensetzung oder einem erfindungsgemäßen kosmetischen (topischen) Produkt sein können, sind:
Konservierungsmittel, vorzugsweise die in US 2006/0089413 genannten, Abrasiva, Antiakne-Mittel und Mittel zu Sebumreduktion, vorzugsweise die in WO 2008/046791 genannten, Mittel gegen Hautalterung, vorzugsweise die in WO 2005/123101 genannten, antibakterielle Mittel, Anticellulitis-Mittel, Antischuppen-Mittel, vorzugsweise die in WO 2008/046795 genannten, entzündungshemmende Mittel, irritationsverhindernde Mittel, Antiirritantien (antiinflammatorische, irritationshemmende und irritationsverhindernde Mittel), vorzugsweise die in WO 2007/042472 und US 2006/0089413 genannten, antimikrobielle Mittel, vorzugsweise die in WO 2005/123101 genannten, Antioxidantien, vorzugsweise die in WO 2005/123101 genannten, Adstringentien, antiseptische Mittel, Antistatika, Binder, Puffer, Trägermaterialien, vorzugsweise die in WO 2005/123101 genannten, Chelatbildnder, vorzugsweise die in WO 2005/123101 genannten, Zellstimulantien, reinigende Mittel, pflegende Mittel, Enthaarungsmittel, oberflächenaktive Substanzen, deodorierende Mittel und Antiperspirantien, vorzugsweise die in WO 2005/123101 genannten, Weichmacher, Emulgatoren, vorzugsweise die in WO 2005/123101 genannten, Enzyme, ätherische Öle, vorzugsweise die in US 2008/0070825 genannten, Insektenrepellentien, vorzugsweise die in WO 2005/123101 genannten, Fasern, Filmbildner, weitere Fixateure, Schaumbildner, Schaumstabilisatoren, Substanzen zum Verhindern des Schäumens, Schaumbooster, Fungizide, gelierende Mittel und gelbildende Mittel, vorzugsweise die in WO 2005/123101 genannten, Haarpflegemittel, Haarverformungsmittel, Haarglättungsmittel, (weitere) Feuchtigkeitsregulatoren (feuchtigkeitsspendende, anfeuchtende und/oder feuchthaltende Substanzen), vorzugsweise die in WO 2005/123101 genannten, Osmolyte, vorzugsweise die in WO 2005/123101 genannten, kompatible Solute, vorzugsweise die in WO 01/76572 und WO 02/15686 genannten, bleichende Mittel, stärkende Mittel, fleckenentfernende Mittel, optisch aufhellende Mittel, imprägnierende Mittel, schmutzabweisende Mittel, reibungsverringernde Mittel, Gleitmittel, Feuchtigkeitscremes, Salben, Trübungsmittel, plastifizierende Mittel, deckfähige Mittel, Politur, Glanzmittel, Polymere, vorzugsweise die in WO 2008/046676 genannten, Pulver, Proteine und Proteinhydrolysate, vorzugsweise die in WO 2005/123101 und WO 2008/046676 genannten, (weitere) rückfettende Mittel, abschleifende Mittel, hautberuhigende Mittel, hautreinigende Mittel, hautpflegende Mittel, hautheilende Mittel (skin repair agents), vorzugsweise enthaltend Cholesterin und/oder Fettsäuren und/oder Ceramide und/oder Pseudoceramide, dabei bevorzugt die in WO 2006/053912 genannten, Hautaufhellungsmittel, vorzugsweise die in WO 2007/110415 genannten, hautschützende Mittel, hauterweichende Mittel, hautkühlende Mittel, vorzugsweise die in WO 2005/123101 genannten, hautwärmende Mittel, vorzugsweise die in WO 2005/123101 genannten, Stabilisatoren, UV-absorbierende Mittel und UV-Filter, vorzugsweise die in WO 2005/123101 genannten, Benzyliden-beta-dicarbonylverbindungen, vorzugsweise die in WO 2005/107692 genannten, alpha-Benzoylzimtsäurenitrile, vorzugsweise die in WO 2006/015954 genannten, AhR-Rezeptor-Antagonisten, vorzugsweise die in WO 2007/128723 und WO 2007/060256 genannten, Waschmittel, Weichspüler, suspendierende Mittel, Hautbräunungsmittel, vorzugsweise die in WO 2006/045760 genannten, Verdickungsmittel, Vitamine, vorzugsweise die in WO 2005/123101 genannten, Öle, Wachse und weitere Fette, vorzugsweise die in WO 2005/123101 genannten, Phospholipide, vorzugsweise die in WO 2005/123101 genannten, Fettsäuren (gesättigte Fettsäuren, ein- oder mehrfach ungesättigte Fettsäuren, α-Hydroxysäuren, Polyhydroxyfettsäuren), vorzugsweise die in WO 2005/123101 genannten, Verflüssiger, Farbstoffe und farbschützende Mittel sowie Pigmente, vorzugsweise die in WO 2005/123101 genannten, Antikorrosiva, weitere Riechstoffe, die nicht Teil der Bestandteile (B) (i) und (B) (ii) sind, vorzugsweise die in S. Arctander, Perfume and Flavor Chemicals, Eigenverlag, Montclair, N.J., 1969 und Surburg, Panten, Common Fragrance and Flavor Materials, 5th Edition, Wiley-VCH, Weinheim 2006 aufgeführten, insbesondere die in US 2008/0070825 explizit genannten, Alkohole und Polyole, vorzugsweise die in WO 2005/123101 genannten, Tenside, vorzugsweise die in WO 2005/123101 genannten, tierische Extrakte, Hefeextrakte, Extrakte von Algen oder Mikroalgen, Elektrolyte, Verflüssiger, organische Lösungsmittel, vorzugsweise die in WO 2005/123101 genannten, oder Silikone und Silikonderivate, vorzugsweise die in WO 2008/046676 genannten.

Bevorzugte erfindungsgemäße kosmetische Produkte als Ausgestaltungen einer erfindungsgemäßen Zusammensetzung sind gewählt aus der Gruppe bestehend aus: Parfüm-Extraits, Eau de Parfums, Eau de Toilettes, Rasierwässer, Eau de Colognes, Preshave-Produkte, Splash-Colognes und parfümierten Erfrischungstüchern sowie die Parfümierung von sauren, alkalischen und neutralen Reinigungsmitteln, vorzugsweise Fußbodenreinigern, Fensterglasreinigern, Geschirrspülmittel, Bad- und Sanitärreinigern, Scheuermilch, festen und flüssigen WC-Reinigern, pulver- und schaumförmigen Teppichreinigern, Textilerfrischern, Bügelhilfen, flüssigen Waschmitteln, pulverförmigen Waschmitteln, Wäschevorbehandlungsmitteln wie Bleichmittel, Einweichmittel und Fleckenentfernern, Wäscheweichspülern, Waschseifen, Waschtabletten, Desinfektionsmitteln, Oberflächendesinfektionsmitteln sowie von Luftverbesserern in flüssiger, gelartiger oder auf einem festen Träger aufgebrachter Form, Aerosolsprays, Wachsen und Polituren wie Möbelpolituren, Fußbodenwachsen, Schuhcremes sowie Körperpflegemitteln, vorzugsweise festen und flüssigen Seifen, Duschgelen, Shampoos, Rasierseifen, Rasierschäumen, Badeölen, kosmetischen Emulsionen vom Öl-in-Wasser-, vom Wasser-in-Öl- und vom Wasser-in-Öl-in-Wasser-Typ, vorzugsweise Hautcremes- und -lotionen, Gesichtscremes und -lotionen, Tagescremes, Nachtcremes, Sonnenschutzcremes-und -lotionen, After-sun-cremes und -lotionen, Handcremes und -lotionen, Fußcremes und -lotionen, Enthaarungscremes und -lotionen, After-shave-Cremes und -lotionen, Bräunungscremes und -lotionen, Haarpflegeprodukten, vorzugsweise Haarsprays, Haargelen, festigenden Haarlotionen, Haarspülungen, permanenten und semipermanenten Haarfärbemitteln, Haarverformungsmitteln wie Kaltwellen und Haarglättungsmitteln, Haarwässern, Haarcremes und -lotionen, Deodorantien und Antiperspirantien, vorzugsweise Achselsprays, Roll-ons, Deosticks, Deocremes, Produkten der dekorativen Kosmetik, vorzugsweise Lidschatten, Nagellacke, Make-ups, Lippenstifte, Mascara sowie von Kerzen, Lampenölen, Räucherstäbchen, Insektiziden und Repellentien.

Bevorzugte erfindungsgemäße Zubereitungen sind kosmetische, insbesondere topische, Produkte, die wie üblich zusammengesetzt sind und dem kosmetischen Lichtschutz, zur Behandlung, der Pflege und der Reinigung der Haut und/oder der Haare oder als Schminkprodukt in der dekorativen Kosmetik dienen. Entsprechend liegen derartige Zubereitungen vor als Reinigungsmittel, vorzugsweise als Seife, Syndet, flüssige Wasch-, Dusch-, und Badepräparat, Hautpflegemittel, vorzugsweise als Emulsion (als Lösung, Dispersion, Suspension; Creme, Lotion oder Milch je nach Herstellungsverfahren und Inhaltsstoffen vom Typ Öl-in-Wasser (O/W), vom Typ Wasser-in-Öl (W/O), oder multiple Emulsion, PIT Emulsion, Emulsionsschaum, Mikro-, Nanoemulsion, Pickering Emulsion), Salbe, Paste, Gel (inklusive Hydro-, Hydrodispersions-, Oleogel), alkoholische oder wässrig/alkoholische Lösung, Öl, Toner, Balsam, Serum, Puder, Wipe, Eau de Toilette, Eau de Cologne, Perfum, Wachs, inklusive der Darreichungsform als Stift, Roll-On, (Pump-)Spray, Aerosol (schäumend, nicht schäumend oder nach-schäumend), Hautpflegemittel (wie oben beschrieben) als Fußpflegemittel (inklusive Keratolytika, Desodorant), als Insekten abwehrendes Mittel, als Sonnenschutzmittel, als Selbstbräunungsmittel und / oder Aftersun-Präparat, Hautpflegemittel als Rasiermittel oder After-Shave, als Haarentfernungsmittel, als Haarpflegemittel, vorzugsweise als Shampoo (inklusive Shampoo für normales Haar, für schnellfettendes Haar, für trockenes, strapaziertes (geschädigtes) Haar, 2-in-1 Shampoo, Anti-Schuppen-Shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (vorzugsweise Gel oder Wachs); Blondiermittel, Haarfärbemittel, vorzugsweise temporäre, direktziehende, semipermanente Haarfärbemittel, permanente Haarfärbemittel), Hautpflegemittel als dekorative Körperpflegemittel, vorzugsweise Nagelpflegemittel (Nagellack und Nagellackentferner), dekorative Kosmetik (z.B. Puder, Lidschatten, Kajalstift, Lippenstift), Deodorant und / oder Antitranspirant.

Erfindungsgemäße kosmetische, vorzugsweise topische, Produkte enthalten

Bestandteil (A) vorzugsweise in einer Menge von 0,25 - 30 Gew.-%, bevorzugt 0,25 - 20 Gew.-%, weiter bevorzugt 0,5 - 15 Gew.-%, besonders bevorzugt 0,5 - 10 Gew.-%, und ganz besonders bevorzugt 1 - 10 Gew.-%,
und/oder
Bestandteil (B) vorzugsweise in einer Menge von 0,15 - 5 Gew.-%, bevorzugt 0,2 - 3 Gew.-%, weiter bevorzugt 0,3 - 3 Gew.-%, besonders bevorzugt 0,3 - 2,5 Gew.-%, und ganz besonders bevorzugt 0,3 - 2 Gew.-%,
jeweils bezogen auf das Gesamtgewicht des kosmetischen Produktes.

Vorzugsweise handelt es sich bei den erfindungsgemäßen Zusammensetzungen um sogenannte "leave-on" - Produkte, d.h. um Produkte, die auf (menschlicher) Haut und/oder (menschlichem) Haar verbleiben und in der Regel nicht abgewaschen werden. Vorzugsweise verbleibt ein erfindungsgemäßes "leave-on" - Produkt (in einer bevorzugten Ausführungsform eines erfindungsgemäßen kosmetischen Produktes) zumindest 15 Minuten oder länger, bevorzugt zumindest 30 Minuten oder länger und weiter bevorzugt zumindest 60 Minuten oder länger auf der (menschlichen) Haut und/oder dem (menschlichen) Haar. Hierzu zählen insbesondere Eau de Parfum, Eau de Toilette, Rasierwasser, (Haut)Cremes, Emulsionen (zur topischen Anwendung), Deosprays, Deo-Roller, Haarsprays, Haarconditioner.

Bevorzugte "leave-on" - Produkte sind gewählt aus der Gruppe bestehend aus:
- Hautcreme- oder -lotion, Gesichtscreme oder -lotion, Tagescreme, Nachtcreme, Sonnenschutzcreme, -spray oder -lotion, After-sun-creme oder -lotion, Handcreme oder -lotion, Fußcreme oder -lotion, After-shave-Creme oder -lotion, Hautaufhellungscreme oder -lotion, Hautbräunungscreme oder -lotion;
- Haarpflegeprodukte, vorzugsweise Haarspray, Haargel, festigenden Haarlotion, Haarwasser, Haarcreme, Haarwachs, Haarlotion, Haarconditioner;
- Deodorantien und Antiperspirantien, vorzugsweise Achselspray, Roll-on, Deostick, Deocreme, und
- Produkte der dekorativen Kosmetik, vorzugsweise Lidschatten, Nagellack, (pflegender) Lippenstift oder Mascara.

Bevorzugt ist eine erfindungsgemäße Zusammensetzung oder ein erfindungsgemäßes kosmetisches Produkt, vorzugsweise in einer der vorstehend als bevorzugt angegebenen Ausgestaltungen, umfassend:
(A) Neopentylglycoldiisononanoat in einer Gesamtmenge von 0,25 - 30 Gew.-%, bevorzugt 0,25 - 20 Gew.-%, weiter bevorzugt 0,5 - 15 Gew.-%, besonders bevorzugt 0,5 - 10 Gew.-%, und ganz besonders bevorzugt 1 - 10 Gew.-%,
   und/oder
(B) einen oder mehrere Riechstoffe in einer Menge von 0,15 - 5 Gew.-%, bevorzugt 0,2 - 3 Gew.-%, weiter bevorzugt 0,3 - 3 Gew.-%, besonders bevorzugt 0,3 - 2,5 Gew.-%, und ganz besonders bevorzugt 0,3 - 2 Gew.-%, wobei vorzugsweise Bestandteil (B)
(B) (i) 2, 3, 4, 5 oder mehr Riechstoffe mit einem Molgewicht im Bereich von 100 g/mol bis 175 g/mol enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
   und/oder
(B) (ii) einen, 2, 3, 4, 5 oder mehr Riechstoffe mit einem Molgewicht von größer oder gleich 190 g/mol (Fondnote) enthält, bevorzugt im Bereich von 190 g/mol bis 300 g/mol, weiter bevorzugt mit einem Molgewicht im Bereich von 195 g/mol bis 290 g/mol, und am meisten bevorzugt im Bereich 200 bis 275 g/mol,
   und/oder
(C) (ii) Dipropylenglycol, Diethylphtalat, Triethylcitrat, Isopropylmyristat und Triacetin, wobei die Gesamtmenge des Bestandteils (C) (ii) bis zu 80 Gew.-%, bevorzugt von 0,5 bis 60 Gew.-%, bevorzugt von 1 bis 50 Gew.-%, weiter bevorzugt von 5 bis 40 Gew.-%, jeweils bezogen auf das Gesamtgewicht des Bestandteils (B) beträgt,
   und/oder
(D) ein oder mehrere Di- oder Triole mit 3 bis 12 C-Atomen, vorzugsweise gewählt aus der Gruppe bestehend aus Glycerin, 1,2-Propylenglykol, 1,2-Butylenglykol, 1,3-Butylenglykol, 1,2-Pentandiol, 1,2-Hexandiol, 1,2-Octandiol und 1,2-Decandiol, in einer Gesamtmenge von 0,2 bis 20 Gew.-%, bevorzugt von 0,5 bis 10 Gew.-%, weiter bevorzugt von 1 bis 5 Gew.-%,
   jeweils bezogen auf das Gesamtgewicht der Zusammensetzung bzw. des Produktes.

Die Gewichtsprozentangaben sind jeweils bezogen auf das Gesamtgewicht der Zusammensetzung. Vorzugsweise sind die zu den Bestandteilen (A), (B), (C) und (D) genannten bevorzugten Gewichtsanteile gleichzeitig eingestellt.

Die vorliegende Erfindung betrifft auch die Verwendung des Bestandteils (A) (wie oben definiert, vorzugsweise in einer oben als besonders bevorzugt angegebenen Ausführungsform) als Mittel zur
- Reduzierung der Verdampfung von Riechstoffen in einer Riechstoffzusammensetzung, insbesondere unmittelbar nach der Applikation;
- zeitlichen Stabilisierung des Geruchsprofils einer Riechstoffzusammensetzung;
- Erhöhung oder zeitlichen Stabilisierung des geruchlichen Impacts (der wahrgenommenen Geruchsstärke) einer Riechstoffzusammensetzung;
- Verlängerung der Haftung von Riechstoffen einer Riechstoffzusammensetzung, insbesondere der Kopfnote, auf Haut und/oder Haar, insbesondere auf menschlicher Haut;
und/oder
- Vermittlung oder Verstärkung eines angenehmen Hautgefühls einer (ethanolischen) Riechstoffzusammensetzung.

Die Erfindung betrifft ferner die Verwendung von Neopentylglycoldiisononanoat als Fixateur für Parfümöle bzw. als Fixateur für (Kopfnoten-) Riechstoffe, wobei vorzugsweise die Kopfnoten-Riechstoffe gewählt sind aus der Liste bestehend aus:
(B) (i) n-Heptanol, trans-9-Decen-1-ol, Campher, alpha-Pinen, beta-Pinen, gamma-Terpinen, alpha-Phellandren, alpha-Terpineol, Borneol, Limonen, 6-Methyl-5-hepten-2-on, n-Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Benzaldehyd, Linalool, Tetrahydrolinalool, Tetrahydrogeraniol, Citral, Neral, Geranial, Benzylalkohol, Benzylacetat, Methylcinnamat, p-Anisaldehyd, Menthol, Isoamylacetat, Isoamylbutyrat, cis-3-Hexenylacetat, trans-3-Hexenylacetat, Hexylacetat, Butylbutyrat, Citronellol, Nerol, Geraniol, 2-Phenylethylalkohol, Methylbenzoat, Methylanthranilat, Styrallylacetat, 2,4-Dimethyl-3-cyclohexencarboxaldehyd (Vertocitral), Acetophenon, p-Methylacetophenon, Zimtalkohol, Zimtaldehyd, 3-Phenylpropanol (Dihydrozimtalkohol), 2-Methyl-1-phenyl-2-propanol (alpha,alpha-Dimethylphenethylcarbinol), Anethol, Anisalkohol, Heliotropin, Acetanisol (p-Methoxyacetophenon) und Agrunitril (3,7-Dimethyl-6-octen-1-nitril).

Die vorliegende Erfindung betrifft ferner ein Verfahren zur
- Reduzierung der Verdampfung von Riechstoffen in einer Riechstoffzusammensetzung, insbesondere unmittelbar nach der Applikation,
- zeitlichen Stabilisierung des Geruchsprofils einer Riechstoffzusammensetzung,
- Erhöhung oder zeitlichen Stabilisierung des geruchlichen Impacts (der wahrgenommenen Geruchsstärke) einer Riechstoffzusammensetzung,
- Verlängerung der Haftung von Riechstoffen einer Riechstoffzusammensetzung, insbesondere der Kopfnote, auf Haut und/oder Haar, insbesondere auf menschlicher Haut,
und/oder
- Vermittlung oder Verstärkung eines angenehmen Hautgefühls einer Riechstoffzusammensetzung,
mit folgendem Schritt:
Mischen von
   (A) Neopentylglycoldiisononanoat, vorzugsweise in einer Bestandteil (B) fixierenden Menge,
      und
   (B) einem oder mehreren Riechstoffen, wobei Bestandteil (B)
   (B) (i) einen, 2, 3, 4, 5 oder mehr Riechstoffe mit einem Molgewicht im Bereich von 100 g/mol bis 175 g/mol (Kopfnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
   wobei vorzugsweise das Massenverhältnis des Bestandteils (A) zur Gesamtmasse des Bestandteils (B) im Bereich von 1 : 20 bis 200 : 1, bevorzugt im Bereich von 1 : 12 bis 100 : 1, liegt, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1,
sowie gegebenenfalls weiteren Bestandteilen, vorzugsweise gewählt aus der Gruppe bestehend aus den (oben bevorzugt genannten) Bestandteilen (C) und/oder (D),
wobei das Massenverhältnis bezogen ist auf die resultierende Zusammensetzung nach dem Mischen.

Die erfindungsgemäßen Zusammensetzungen lassen sich auf einfache Weise herstellen durch Mischen der Einzelkomponenten der Bestandteile (A) und (B) sowie gegebenenfalls (C) und/oder (D). Dabei ist die Reihenfolge der Kontaktierung der Einzelkomponenten beziehungsweise Bestandteile nicht kritisch und kann variiert werden.

Für die erfindungsgemäßen Verfahren und Verwendungen gelten die obigen Angaben zu bevorzugten erfindungsgemäßen Zusammensetzungen und kosmetischen (topischen) Produkten entsprechend.

Die vorliegende Erfindung betrifft auch ein Verfahren zur
- Vermittlung, Verstärkung oder Modifizierung eines Geruchs auf (menschlicher) Haut,
- Verlängerung der Haftung von Riechstoffen einer Riechstoffzusammensetzung, insbesondere der Kopfnote, auf Haut und/oder Haar, insbesondere auf menschlicher Haut,
und/oder
- Vermittlung oder Verstärkung eines angenehmen Hautgefühls einer Riechstoffzusammensetzung,
mit folgendem Schritt:
- Applizieren einer erfindungsgemäßen Zusammensetzung (bevorzugt einer Zusammensetzung, die oben als bevorzugt angegeben ist) oder eines erfindungsgemäßen (topischen) kosmetischen Produktes (bevorzugt eines Produktes, das oben als bevorzugt angegeben ist) auf die (menschliche) Haut.

Ferner betrifft die Erfindung ein Riechstoff-Fixierungsmittel - Kit (Zusammenstellung), umfassend
eine erste Komponente umfassend oder bestehend aus
(A) Neopentylglycoldiisononanoat,
   und
   eine zweite Komponente umfassend oder bestehend aus
(B) eine oder mehreren Riechstoffen, vorzugsweise einer Riechstoffmischung umfassend 2, 3, 4, 5, 6, 7, 8, 9, 10 oder mehr Riechstoffe,
wobei Bestandteil (B)
(B) (i) einen, 2, 3, 4, 5 oder mehr Riechstoffe mit einem Molgewicht im Bereich von 100 g/mol bis 175 g/mol (Kopfnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
wobei vorzugsweise Bestandteil (B) gewählt ist gemäß den oben bevorzugt bezeichneten Ausführungsformen.

Es hat sich darüberhinaus gezeigt, dass Bestandteil (A) sich nicht nur wie oben bereits beschrieben als Fixateur für Riechstoffe eignet, sondern ebenfalls als Lösemittel für kosmetische UV-Lichtschutzfilter, insbesondere für lipophile (kristalline) UV-Lichtschutzfilter. Daneben wurde gefunden, dass Bestandteil (A) aufgrund der gleichmäßigen Verteilung der (vorzugsweise lipophilen) kosmetischen UV-Filter eine SPF-Erhöhung bewirkt, d.h. einen höheren Lichtschutzfaktor und damit eine höhere UV-Schutzleistung. Geeignete und bevorzugte UV-Filter sind dabei die in WO 2005/123101 genannten.

Darüberhinaus wurde gefunden, dass Bestandteil (A) den Glanz von menschlichem und tierischem Haar verbessert. Insoweit sind gemäß eines Aspekts der vorliegenden Erfindung Haarpflegemittel bevorzugt, vorzugsweise gewählt aus der Gruppe bestehend aus Shampoo, dabei wiederum bevorzugt sind Shampoo für normales Haar, für schnellfettendes Haar, für trockenes, strapaziertes (geschädigtes) Haar, 2-in-1 Shampoo, Anti-Schuppen-Shampoo, Babyshampoo, Shampoo für trockene Kopfhaut, Shampookonzentrat), Conditioner, Haarkur, Haarwasser, Haarspülung, Frisiercreme, Pomade, Dauerwell- und Fixierungsmittel, Haarglättungsmittel (Entkräuselungsmittel, Relaxer), Haarfestiger, Styling-Hilfe (vorzugsweise Gel oder Wachs); Haarfärbemittel.

Darüberhinaus wurde gefunden, dass Bestandteil (A) hautfeuchteregulierend wirkt, indem der Hautfeuchtegehalt (menschlicher) Haut verbessert und der transepidermale Wasserverlust (TEWL) reduziert wird, ohne dabei okkludierende Eigenschaften zu besitzen.

### Weitere Eigenschaften des Bestandteils (A) in erfindungsgemäßen Zusammensetzungen und erfindungsgemäßen kosmetischen Produkten:

- hohe Hautabsorption
- bei 20°C geringe Viskosität
- gutes (kosmetisches) Feuchthaltemittel (moisturizer)
- gute Rückfettungseigenschaften, z.B. als Bestandteil von Deodorantien, Lippenstiften oder Shampoos
- sehr gute Spreitfähigkeit
- geringe Polarität
- sehr gute Löslichkeit in wässrig-alkoholischen kosmetischen Produkten
- bietet einen Wasser abweisenden Effekt
- hochbeständig gegen Oxidation
- schützt die (menschliche) Haut vor dem Austrocknen
- nicht okkludierend
- verleiht (menschlichen) Haaren Glanz, Elastizität und Weichheit
- gibt ein angenehm weiches, glattes und geschmeidiges Hautgefühl.

Aufgrund der genannten Eigenschaften des Bestandteils (A) in erfindungsgemäßen Zusammensetzungen und erfindungsgemäßen kosmetischen Produkten sind folgende Formulierungsarten und Anwendungsbereiche besonders bevorzugt:
- Emulsionen, insbesondere wegen ihrer hervorragenden Spreitfähigkeit,
- Haarpflegeprodukte, insbesondere weil sie den Haaren Glanz, Elastizität und Weichheit verleihen,
- Seifen, weil sie der Entfettung der (menschlichen) Haut entgegenwirken und die Entfettung vermindern oder verhindern,
- Deodorantien, Antiperspirantien, Fettstifte und Lippenstifte, da sie rückfettend und hautfreundlich sind,
- Ethanolische oder wässrig-ethanolische kosmetische Produkte, wie ethanolische Riechstoffmischungen, Eau de Toilettes oder Deodorantien, da sie durch Ethanol hervorgerufene Hautirritationen reduzieren oder verhindern.

Die folgenden Beispiele erläutern die Erfindung; sofern nicht anders angegeben beziehen sich Anteile und Prozente auf das Gewicht.

### Beispiele

Verwendete Abkürzungen: SPF = Lichtschutzfaktor (sun protection factor); MW = Molgewicht; Ret. = Retentionserhöhung; DPG = Dipropylenglycol

### Test 1: Untersuchung der reduzierten Freisetzung von Riechstoffen aus einem Riechstoffgemisch enthaltend ein Massenäguivalent Neopentylglycoldiisononanoat

### Testdurchführung:

50 µl eines Riechstoffgemisches enthaltend die in der nachfolgenden Tabelle aufgelisteten Riechstoffe wurden zu 0,5 % in EtOH gelöst. Einem Aliquot der ethanolischen Lösung wurde ein gleiche Menge an Neopentylglycoldiisononanoat zugesetzt, d.h. das Gewichtsverhältnis der Gesamtmasse an Riechstoffen und Neopentylglycoldiisononanoat lag bei 1 : 1.

Die beiden Lösungen wurden jeweils auf eine definierte Fläche Riechstreifen (2 cm²) aufgetragen und 15 min bei 22 °C äqulibriert. Anschließend wurden die Riechstreifen mit jeweils 4 ml Aceton extrahiert und mit 100 µg Diphenylmethan als Internen Standard (IS) versetzt. Die Proben wurden im Anschluss per GC/MS vermessen, die auf den Riechstoffen verbliebene Menge an Riechstoffen mittels IS-Methode quantifiziert.

Die nachfolgende Tabelle zeigt die Ergebnisse der durchgeführten quantitativen Analysen und die daraus berechnete prozentuale Erhöhung der Retention des jeweiligen Parfümerie-Riechstoffes.

| **Riechstoff** | **MW** | **GC-Counts mit (A)** | **GC-Counts ohne (A)** | **Ret. %** | **Bestandteil** | |
|---|---|---|---|---|---|---|
| n-Heptanol | 118,23 | 233939 | 24436 | 857 | (B) (i) | |
| 2-Phenylethylalkohol | 122,17 | 2061675 | 1368972 | 51 | (B) (i) | |
| Anisaldehyd | 136,15 | 3073838 | 2145579 | 43 | (B) (i) | |
| Agrunitril | 151,25 | 2069626 | 877864 | 136 | (B) (i) | |
| Campher | 152,24 | 239895 | 27060 | 787 | (B) (i) | |
| Geraniol | 154,25 | 2278603 | 1654064 | 138 | (B) (i) | |
| Linalool | 154,25 | 676146 | 106166 | 537 | (B) (i) | |
| Dihydromyrcenol | 156,27 | 609603 | 72942 | 736 | (B) (i) | |
| γ-Decalacton | 170,25 | 3948156 | 3576703 | 10 | (B) (i) | |
| β-Ionon | 192,30 | 4447058 | 3543135 | 26 | | (B) (ii) |
| Lilial® | 204,31 | 5471716 | 4864188 | 13 | | (B) (ii) |
| Benzylbenzoat | 212,25 | 6493182 | 6405967 | 1 | | (B) (ii) |
| Hexylsalicylat | 222,28 | 4395718 | 4006701 | 10 | | (B) (ii) |
| Cedrol | 222,40 | 6647973 | 5706666 | 16 | | (B) (ii) |
| Macrolide® | 240,38 | 6052651 | 5872871 | 3 | | (B) (ii) |

Agrunitril = 3,7-Dimethyl-6-octen-1-nitril; Lilial® = 2-Methyl-3-(4-tert-butylphenyl)propanal; Macrolide® = Oxacyclohexadecan-2-on.

Die Ergebnisse der quantitativen Untersuchungen belegen eindeutig die fixierenden Eigenschaften von Neopentylglycoldiisononanoat. Diese zeigen deutlich, dass die Abdampfrate von Riechstoffen, insbesondere von Kopfnoten-Riechstoffen, in Anwesenheit von Neopentylglycoldiisononanoat signifikant reduziert werden kann, wodurch die fixierenden Eigenschaften dieses Esters für Riechstoffe eindeutig belegt werden konnte. Diese analytischen Ergebnisse konnten sensorisch nachvollzogen werden.

**Formulierungsbeispiele kosmetischer Produkte enthaltend eine erfindungsgemäße Zusammensetzung**

| | |
|---|---|
| **1 = Aerosol Deo-Spray** | **7 = Deo Roll-on Emulsion (Antiperspirant)** |
| **2 = Aerosol Haarspray** | **8 = Hautkühlende Körpercreme** |
| **3= Hautöl** | **9 = Sonnenschutzcreme mit SPF 20** |
| **4 = Eau de Toilette** | **10 = Pflegendes After Sun Spray** |
| **5 = Sonnenschutzöl mit SPF 6** | **11 = Tagescreme** |
| **6 = Badeöl, selbstemulgierend** | **12 = Nachtcreme W/O** |

Die jeweilige Zusammensetzung der in den nachfolgenden kosmetischen Produkten 1 bis 12 verwendeten Parfümöle P1 bzw. P2 ist weiter unten beschrieben.

| **Material** | **INCI-Name** | **Gew.-%** | | | | | | | | | | | |
|---|---|---|---|---|---|---|---|---|---|---|---|---|---|
| | | **1** | **2** | **3** | **4** | **5** | **6** | **7** | **8** | **9** | **10** | **11** | **12** |
| Neopentylglycoldiisono nanoat | 2,2-Dimethyl-1,3-propandiol diisononaoate | **0,5** | **0,3** | **10,0** | **1,0** | **6,0** | **5,0** | **2,5** | **7,0** | **2,0** | **6,0** | **4,5** | **7,0** |
| (-) alpha Bisabolol, natürlich | Bisabolol | 0,1 | | | | | | | | | 0,2 | | 0,3 |
| Abil 350 | Dimethicone | | | | | | | | 2,0 | | | | |
| Aliminiumstearat | Aluminium Stearate | | | | | | | | | | | | 1,2 |
| Avocado-Öl | Persea Gratissima (Avocado) Oil | | | | | | | | | | | 3,0 | |
| L-Arginin | Arginine | | | | | | | | | 0,5 | | | |
| Carbopol Ultrez-10 | Carbomer | | | | | | | | 0,2 | | 0,2 | | |
| Kosmetikfarbe, braun, Pulver | Color | | | | | | | | | | | 4,0 | |
| Covi-Ox T-70 | Tocopherol | | | 0,1 | | | | | | | | 0,1 | |
| Cutina GMS V | Glyceryl Stearate | | | | | | | | 2,0 | | 2,0 | | |
| DC 9701 Pulver | Dimethicone/Vinyl Dimethicone Crosspolymer | | | | | | | | | | | | |
| Dehydol LS3 Deo | Laureth-3 | | | | | | 8,0 | | | | | | |
| Deolite | Dimethyl Phenylpropanol Pentylene Glycol | | | | | | | 0,2 | | | | | |
| Diammoniumcitrat | Diammonium Citrate | | | | | | | | | | | | |
| Dow Corning 246 fluid | Cyclohexasiloxane | | | | | | | 1,0 | | | | 2,0 | |
| Dow Corning 345 fluid | Cyclomethicone | | | | | | | | | | | | |
| D-Panthenol 75 L | Panthenol | | | | | | | | | | | 1,0 | |
| Dracorin® 100 S.E.P. | Glyceryl Stearate, PEG-100 Stearate | | | | | | | 0,5 | | | | | |
| Dracorin® CE | Glyceryl Stearate/Citrate | | | | | | | | | 2,0 | | | |
| Dracorin® GOC | Glyceryl Oleate Citrate Caprylic Capric Triglyceride | | | | | | | 2,0 | | | | 2,0 | |
| Drago-Beta-Glucan | Water (Aqua), Butylene Glycol, Glycerin, Avena Sativa (Oat) Kernel Extract | | | | | | | | | | 2,0 | | |
| DragoCalm® | Water, Glycerin, Avena Sativa (Oat Kernel Extract) | | | | | | | | | | 1,0 | | |
| Dragocide® Liquid | Phenoxyethanol, Methylparaben, Ethylparaben, Butylparaben, Propylparaben, Isobutylparaben | | | | | | | 0,8 | 0,8 | | | | 0,8 |
| Dragoderm® | Glycerin, Triticum Vulgare (Wheat) Gluten, Water (Aqua) | | | | | | | | | | 2,0 | 2,0 | |
| Dragosan W/O P | Sorbitan Isostearate, Hydrogenated Castor Oil, Ceresin, Beeswax (Cera Alba) | | | | | | | | | | | | 8,0 |
| Dragosantol® 100 | Bisabolol | | | 0,2 | | 0,3 | | 0,2 | | | | | |
| Dragosine® | Carnosine | | | | | | | | | | 0,2 | | |
| Dragoxat® 89 | Ethylhexyl Isononanoate | | | 15,0 | | 4,0 | | | | 3,0 | 4,0 | | 5,0 |
| EDTA | Disodium EDTA | | | | | | | | | 0,1 | 0,1 | | |
| Emulsiphos® | Potassium Cetyl Phosphate, Hydrogenated Palm Glycerides | | | | | | | | 2,0 | | 2,0 | | |
| Ethanol | Ethanol | 28,0 | 45,2 | | Ad 100 | | | | 5,0 | | | 5,0 | |
| Farbe, braun, E 172 + E 171 | Color | | | | | | | | | | 4,0 | | |
| Parfümöl "P1" | Perfume | 1,0 | 0,3 | 0,3 | 10,0 | 0,5 | 2,0 | | | | | | |
| Parfümöl "P2" | Perfume | | | | | | | 0,4 | 0,3 | 0,4 | 0,3 | 0,5 | 0,3 |
| Frescolat® ML | Menthyl Lactate | | | | | | | 0,5 | 0,3 | | | 0,5 | |
| Fruitapone® Orange B | Propylene Glycol, Water (Aqua), Citric Acid, Citrus Aurantium Dulcis (Orange) Juice, Trideceth-9, Bisabolol | | | | | | | | 1,0 | | | | |
| Glycerin 85% in Wasser | Glycerin | | | | | | | | 2,0 | | 3,0 | | 3,0 |
| Glycerin | Glycerin | | | | | | | 4,0 | | | | 4,0 | |
| Hydrolite®-5 | Pentylene Glycol (1,2-Pentandiol) | | | | | | | | | 5,0 | | 5,0 | |
| Hydroviton®-24 | Water, Pentylene Glycol, Glycerin, Lactic Acid, Sodium Lactate, Serine, Urea, Sorbitol, Sodium Chloride, Allantoin | | | | | | | | | | 1,0 | | 2,0 |
| Iso Adipat | Diisopropyl Adipate | | | | | | | | | | 1,0 | | |
| Isodragol® | Triisononanoin | | | | | | | 1,0 | | | | | |
| Jojoba-Öl | Simmondsia Chinensis (Jojoba) Seed Oil | | | | | | | | | | | | 2,0 |
| Keltrol CG RD | Xanthan Gum | | | | | | | | 0,1 | 0,1 | 0,2 | | |
| Lanette O | Cetearyl Alcohol | | | | | | | | 3,0 | 2,0 | 3,0 | | |
| Luviskol K30 | PVP | | 4,5 | | | | | | | | | | |
| Mineralöl | Mineral Oil | | | | | 64,2 | | | | | | | 8,0 |
| Neo Heliopan® 303 | Octocrylene | | | | | | | | | 5,0 | | | |
| Neo Heliopan® 357 | Butylmethoxydibenzoylmethane | | | | | 1,5 | | | | 4,0 | 1,5 | | |
| Neo Heliopan® E 1000 | Isoamyl p.Methoxycinnamate | | | | | | | | | | 5,0 | | |
| Neo Heliopan® Hydro, 25% in Wasser, neutralisiert mit L-Arginin | Phenylbenzimidazole Sulfonic Acid | | | | | | | | | 8,0 | 3,3 | | |
| Neo Heliopan® MBC | 4-Methylbenzylidene Camphor | | | | | 1,5 | | | | | | | |
| Neo Heliopan® OS | Ethylhexyl Salicylate | | | | | | | | | 5,0 | | | |
| Neutralöl | Caprylic/Capric Triglyceride | | | Ad 100 | | 22,0 | Ad 100 | 3,5 | | | | 5,0 | |
| Ozokerit Wax 2389 | Ozokerite | | | | | | | | | | | | 2,0 |
| Pemulen TR-2 | Acrylates/C10-30 Alkyl Acrylate Crosspolymer | | | | | | | 0,3 | | | | 0,3 | |
| Polyglycol 400 | PEG-8 | | 0,1 | | | | | | | | | | |
| Kaliumsorbat | Potassium Sorbate | | | | | | | | | | | 0,1 | |
| Propan-Butan 2,7 bar | Propane, Butane | Ad 100 | Ad 100 | | | | | | | | | | |
| Propyleneglycol | Propylene Glycol | | | | | | | | 3,0 | 4,0 | | | |
| Rezal 36 GP | Aluminium Zirconium Tetrachlorohydrex GLY | | | | | | | 5,0 | | | | | |
| Natriumchlorid | Sodium Chloride | | | | | | | | | | | | 1,0 |
| NaOH, 10% in Wasser | Sodium Hydroxide | | | | | | | 0,6 | 0,5 | | | | 0,4 |
| Softisan 100 | Hydrogenated Coco Glycerides | | | | | | | | | 1,5 | | | |
| Solubilisator | PEG-40Hydrogenated Castor Oil, Trideceth-9, Propylene Glycol, Water (Aqua) | | | | 1,0 | | | | | | | | |
| Squalan, pflanzlich | Squalane | | | | | | | | | | 3,0 | | |
| Mandelöl | Prunus Dulcis | | | 20,0 | | | | | | | | | |
| SymCalmin® | Pentylene Glycol, Butylene Glycol, Hydroxyphenyl Propamidobenzoic Acid | | | | | | | | | | | | |
| SymClariol® | Decylene Glycol | 0,5 | | | | | | | | | | | |
| SymDeo® MPP | Dimethyl Phenylbutanol | 0,5 | | | | | | | | | | | |
| SymDiol® 68 | 1,2 Hexanediol, Caprylyl Glycol (1:1) | | | | | | | | | | 1,0 | | |
| SymGlucan® | Water (Aqua) Glycerin, Beta Glucan | | | | | | | | | | | 1,0 | |
| SymMollient® W/S | Trideceth-9, PEG-5 Isononanoate | | | | 1,0 | | | | | | | | |
| SymRelief® | Bisabolol, Zingiber Officinale (Ginger) Root Extract | | | | | | | | | | | 0,2 | |
| SymRepair® | Hexyldecanol, Bisabolol, Cetylhydroxyproline Palmitamide, Stearic Acid, Brassica Campestris (Rapeseed Sterols) | | | | | | | | | | 2,0 | 3,0 | |
| SymVital™ | Aloe Barbadensis Leaf Juice Powder, Magnesium Ascorbyl Phosphate, Rubus Idaeus (Raspberry) Leaf Extract | | | | | | | | 0,1 | | | | |
| Triethanolamin | Triethanolamine | | | | | | | | | | 0,9 | 0,3 | |
| Vitamin-E-acetat | Tocopherol Acetate | | | 0,5 | | | | | | 0,5 | | | 0,2 |
| Wasser | Water (Aqua) | | | | 8,0 | | | Ad 100 | Ad 100 | Ad 100 | Ad 100 | Ad 100 | Ad 100 |

Das in den Formulierungsbeispielen 1 bis 6 eingesetzte Parfümöl "P1" mit Rosengeruch hatte folgende Zusammensetzung:

| Komponente / NAME | Gew.-teile |
|---|---|
| Acetophenon, 10%ig in DPG | 10,00 |
| n-Undecanal | 5,00 |
| Gamma-Undecalacton | 15,00 |
| Allylamylglycolat, 10%ig in DPG | 20,00 |
| Amylsalicylat | 25,00 |
| Benzylacetat | 60,00 |
| Citronellol | 80,00 |
| D-Limonen | 50,00 |
| trans-9-Decen-1-ol | 15,00 |
| Dihydromyrcenol | 50,00 |
| Dimethylbenzylcarbinylacetat | 30,00 |
| Diphenyloxid | 5,00 |
| GALAXOLIDE® | 20,00 |
| Geraniol | 40,00 |
| Nerol | 20,00 |
| Geraniumöl | 15,00 |
| Hexenol cis-3, 10%ig in DPG | 5,00 |
| Hexenylsalicylat cis-3 | 20,00 |
| Indol, 10%ig in DPG | 10,00 |
| Alpha-Ionon | 15,00 |
| Beta-Ionon | 5,00 |
| Lilial® (2-Methyl-3-(4-tert-butyl-phenyl)propanal) | 60,00 |
| Linalool | 40,00 |
| Methylphenylacetat | 10,00 |
| Phenylethylalkohol | 245,00 |
| Styrolylacetat | 20,00 |
| Terpineol | 30,00 |
| Tetrahydrolinalool | 50,00 |
| Ethylenbrassylat | 30,00 |
| Summe: | 1.000,00 |

Das in den Formulierungsbeispielen 7 bis 12 eingesetzte Parfümöl "P2" mit einem weißen Blütenduft und Moschusnote hatte folgende Zusammensetzung:

| Komponente / NAME | Gew.-teile |
|---|---|
| Benzylacetat | 60,00 |
| Citronellylacetat | 60,00 |
| Cyclamenaldehyd (2-Methyl-3-(4-isopropylphenyl)propanal | 20,00 |
| Dipropylenglycol | 60,00 |
| Ethyllinalool | 40,00 |
| Florol (2-Isobutyl-4-methyltetrahydro-2H-pyran-4-ol) | 30,00 |
| Globanone [(E/Z)-8-Cyclohexadecen-1-on] | 100,00 |
| Ethylenbrassylat | 80,00 |
| Hedione® (Methyldihydrojasmonat) | 140,00 |
| Hexenylsalicylat, cis-3 | 10,00 |
| Vertocitral (2,4-dimethyl-3-cyclohexencarboxaldehyd) | 5,00 |
| Hydratropaaldehyd, 10%ig in DPG | 5,00 |
| Isodamascon (1-(2,4,4-Trimethyl-2-cyclohexen-1-yl)-2-buten-1-on), 10%ig in DPG | 5,00 |
| Cyclohexadecanon | 40,00 |
| Jacinthaflor (2-Methyl-4-phenyl-1,3-dioxolan) | 10,00 |
| Cis-Jasmon, 10%ig in DPG | 20,00 |
| Linalool | 50,00 |
| Linalylacetat | 30,00 |
| Methylbenzoat, 10%ig in DPG | 25,00 |
| para-Methylkresol, 10%ig in DPG | 10,00 |
| Nerol | 20,00 |
| Phenylpropylaldehyd | 5,00 |
| 2-Phenylethylalkohol | 82,00 |
| Tetrahydrogeraniol | 13,00 |
| 2,2-Dimethyl-3-cyclohexyl-1-propanol | 40,00 |
| Tonalide® | 40,00 |
| Gesamt: | 1.000,00 |

## Patentansprüche

1. Zusammensetzung umfassend oder bestehend aus
(A) Neopentylglycoldiisononanoat,
und
(B) einem oder mehreren Riechstoffen, vorzugsweise einer Riechstoffmischung umfassend 2, 3, 4, 5, 6, 7, 8, 9, 10 oder mehr Riechstoffe,
wobei Bestandteil (B)
(B) (i) einen, 2, 3, 4, 5 oder mehr Riechstoffe mit einem Molgewicht im Bereich von 100 g/mol bis 175 g/mol (Kopfnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
und
wobei Bestandteil (A) vorzugsweise in einer für den, mehrere oder sämtliche Riechstoffe des Bestandteils (B), bevorzugt in einer für den, mehrere oder sämtliche Riechstoffe des Bestandteils (B) (i) fixierenden Menge, enthalten ist.

2. Zusammensetzung nach Anspruch 1, **dadurch gekennzeichnet, dass** das Massenverhältnis des Bestandteils (A) zur Gesamtmasse des Bestandteils (B) im Bereich von 1 : 20 bis 200 : 1 liegt, bevorzugt im Bereich von 1 : 12 bis 100 : 1, weiter bevorzugt im Bereich von 1 : 6 bis 50 : 1, besonders bevorzugt im Bereich von 1 : 5 bis 33 : 1, und ganz besonders bevorzugt im Bereich von 1 : 2 bis 25 : 1.

3. Zusammensetzung nach Anspruch 1 oder 2, **dadurch gekennzeichnet, dass** Bestandteil (B) zusätzlich
(B) (ii) einen, 2, 3, 4, 5 oder mehr Riechstoffe mit einem Molgewicht von größer oder gleich 190 g/mol (Fondnote) enthält, vorzugsweise mit einem Molgewicht im Bereich von 190 g/mol bis 300 g/mol, weiter bevorzugt mit einem Molgewicht im Bereich von 195 g/mol bis 290 g/mol, und am meisten bevorzugt im Bereich 200 bis 275 g/mol.

4. Zusammensetzung nach einem der vorhergehenden Ansprüche, **dadurch gekennzeichnet, dass** der, mehrere oder sämtliche Riechstoffe des Bestandteils (B) (i) (Kopfnote) ausgewählt sind aus der Gruppe bestehend aus:
(B) (i) n-Heptanol, trans-9-Decen-1-ol, Campher, alpha-Pinen, beta-Pinen, gamma-Terpinen, alpha-Phellandren, alpha-Terpineol, Borneol, Limonen, 6-Methyl-5-hepten-2-on, n-Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Benzaldehyd, Linalool, Tetrahydrolinalool, Tetrahydrogeraniol, Citral, Neral, Geranial, Benzylalkohol, Benzylacetat, Methylcinnamat, p-Anisaldehyd, Menthol, Isoamylacetat, Isoamylbutyrat, cis-3-Hexenylacetat, trans-3-Hexenylacetat, Hexylacetat, Butylbutyrat, Citronellol, Nerol, Geraniol, 2-Phenylethylalkohol, Methylbenzoat, Methylanthranilat, Styrallylacetat, 2,4-Dimethyl-3-cyclohexencarboxaldehyd (Vertocitral), Acetophenon, p-Methylacetophenon, Zimtalkohol, Zimtaldehyd, 3-Phenylpropanol (Dihydrozimtalkohol), 2-Methyl-1-phenyl-2-propanol (alpha,alpha-Dimethylphenethylcarbinol), Anethol, Anisalkohol, Heliotropin, Acetanisol (p-Methoxyacetophenon) und Agrunitril (3,7-Dimethyl-6-octen-1-nitril).

5. Zusammensetzung nach einem der vorhergehenden Ansprüche, **dadurch gekennzeichnet, dass** der, mehrere oder sämtliche Riechstoffe des Bestandteils (B) (ii) ausgewählt sind aus der Gruppe bestehend aus:
- Moschusriechstoffen,
alpha-n-Amylzimtaldehyd (MW = 202,30), alpha-iso-Amylzimtaldehyd (MW = 202,30), alpha-n-Hexylzimtaldehyd (MW = 216,32), alpha-iso-Hexylzimtaldehyd (MW = 216,32), Benzylsalicylat (MW = 228,25), cis-3-Hexenylsalicylat (MW = 220,27), Isoamylsalicylat (MW = 208,26), Hexylsalicylat (MW = 222,28), 2-Methyl-3-(4-tert-butylphenyl)propanal (MW = 204,31; Lilial®), 2-Methyl-3-(4-isopropylphenyl)propanal (MW = 190,28, Cyclamenaldehyd), 2,3,8,8-Tetramethyl-1,2,3,4,5,6,7,8-octahydro-2-naphthalenylmethylketon (MW = 234,38, Iso E Super®), 1-[1,2,3,4,6,7,8,8a-octahydro-1,2,8,8-tetramethylnaphtalen-2-yl]ethanon (MW = 234,38, Iso E Super®), Methyldihydrojasmonat (MW = 226,32, Hedione®), Linalylacetat (MW = 196.29), Ethyllinalylacetat (MW = 210,31), Nerolidol (MW = 222,37), Farnesol (MW = 222,37), Cedrylmethylether (MW = 236,40, Cedramber), Cedrylmethylketon (MW = 246,39), Cedrylacetat (MW = 264,41), (4aR,5R,7aS,9R)-Octahydro-2,2,5,8,8,9a-hexamethyl-4H-4a,9-Methanoazuleno(5,6-d) 1,3-dioxol) (MW = 278,44, Ambrocenide®), Hexahydro-1',1',5',5'-tetramethyl-spiro[1,3-dioxolan,2,8' (5'H)-[2H-2,4a]-methanonaphthalin (MW = 264,41 Ethylendioxi-3H-isolongifolan, Ysamber® K), 2-Methyl-4-(2,2,3-trimethyl-3-cyclopenten-1-yl)butanol (MW = 196,34, Brahmanol), 5-(2,2,3-Trimethyl-3-cyclopenten-1-yl)-3-methylpentan-2-ol (MW = 210,36, Sandalore®), 2-Ethyl-4-(2,2,3-trimethyl-3-cyclopenten-1-yl)-2-buten-1-ol (MW = 208,35, Sandranol®), 3-Methyl-5-(2,2,3-trimethyl-3-cyclopenten-1-yl)-4-penten-2-ol (MW = 208,35, Ebanol®), 3,3-Dimethyl-5-(2,2,3-trimethyl-3-cyclopenten-1-yl)-4-penten-2-ol (MW = 222,37, Polysantol®), 3-Isocamphylcyclohexanol (MW = 236,40, Sandel 80®),1-(2,2,6-Trimethylcyclohexyl)hexan-3-ol (MW = 226,41, Timberol®), Cyclododecylmethylether (MW = 198,35, Palisandin), (Ethoxymethoxy)cyclododecan (MW = 242,41, Boisambrene forte®), 1-Methyl-4-(4-methyl-3-penten-1-yl)-3-cyclohexencarboxaldehyd (MW = 206,33, Precyclemone B®), 4-(4-Hydroxy-4-methylpentyl)-3-cyclohexencarboxaldehyd (MW = 210,32, Lyral®), 2-Methyl-4-(2,2,6-trimethyl-1-cyclohexen-1-yl)-2-butenal (MW = 206,33, Boronal), Decahydro-beta-naphthylacetat (MW = 196,29), Allyl-3-cyclohexylpropionat (MW = 196,29), Allylcyclohexyloxyacetat (MW = 198,26, Isoananat®), Citraldiethylacetal (MW = 226,36), Benzylbenzoat (MW = 212,25), Benzylcinnamat (MW = 238,29), 3a,6,6,9a-Tetramethyldodecahydronaphtho[2,1-*b*]furan (MW = 236,40, Ambroxid®), alpha-Iron (MW = 206,33), beta-Iron (MW = 206,33), alpha-n-Methylionon (MW = 206,33), beta-n-Methylionon (MW = 206,33), alpha-Isomethylionon (MW = 206,33), beta-Isomethylionon (MW = 206,33) und Allylionon (MW 232,35). 2,3,8,8-Tetramethyl-1,2,3,4,5,6,7,8-octahydro-2-naphthalenylmethylketon (MW = 234,38), 1-[1,2,3,4,6,7,8,8a-octahydro-1,2,8,8-tetramethylnaphtalen-2-yl]ethanon (MW = 234,38), Isobornylacetat (MW = 196,29), alpha-Ionon (MW = 192,30), beta-Ionon (MW = 192,30), gamma-Ionon (MW = 192,30), alpha-Damascon (MW = 192,30), beta-Damascon (MW = 192,30), delta-Damascon (MW = 192,30), 1-(2,4,4-Trimethyl-2-cyclohexen-1-yl)-2-buten-1-on (MW = 192,30, Isodamascon), Cedrol (MW = 222,40), gamma-Dodecalacton (MW = 198,30), 2-Methyl-3-(3,4-methylendioxyphenyl)propanal (MW = 192,22, Helional) und Methyldihydrojasmonat (MW = 226,31).

6. Zusammensetzung nach einem der vorangehenden Ansprüche, als zusätzlichen Bestandteil enthaltend eine wirksame Menge
(C) eines oder mehrerer kosmetisch akzeptabler Lösungsvermittler für Bestandteil (B), vorzugsweise gewählt aus der Gruppe bestehend aus
(i) Ethanol
und
(ii) Dipropylenglycol, Diethylphtalat, Triethylcitrat, Isopropylmyristat und Triacetin, dabei bevorzugt ist Dipropylenglycol.

7. Zusammensetzung nach einem der vorangehenden Ansprüche, als zusätzlichen Bestandteil enthaltend
(D) ein oder mehrere Di- oder Triole mit 3 bis 12 C-Atomen, vorzugsweise gewählt aus der Gruppe bestehend aus Glycerin, 1,2-Propylenglykol, 1,2-Butylenglykol, 1,3-Butylenglykol und Alkandiolen mit 5 bis 12 C-Atomen.

8. Kosmetisches (topisches) Produkt enthaltend eine (sensorisch wirksame Menge einer) Zusammensetzung wie in einem der vorangehenden Ansprüche definiert.

9. Zusammensetzung nach einem der Ansprüche 1 - 7 oder kosmetisches, vorzugsweise topisches, Produkt, nach Anspruch 8, enthaltend
(A) Neopentylglycoldüsononanoat in einer Gesamtmenge von 0,25 - 30 Gew.-%, bevorzugt 0,25 - 20 Gew.-%, weiter bevorzugt 0,5 - 15 Gew.-%, besonders bevorzugt 0,5 - 10 Gew.-%, und ganz besonders bevorzugt 1 - 10 Gew.-%,
und/oder
(B) einen oder mehrere Riechstoffe in einer Menge von 0,15 - 5 Gew.-%, bevorzugt 0,2 - 3 Gew.-%, weiter bevorzugt 0,3 - 3 Gew.-%, besonders bevorzugt 0,3 - 2,5 Gew.-%, und ganz besonders bevorzugt 0,3 - 2 Gew.-%, wobei vorzugsweise Bestandteil (B)
- (B) (i) 2, 3, 4, 5 oder mehr Riechstoffe mit einem Molgewicht im Bereich von 100 g/mol bis 175 g/mol (Topnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
und/oder
- (B) (ii) einen, 2, 3, 4, 5 oder mehr Riechstoffe mit einem Molgewicht von größer oder gleich 190 g/mol (Fondnote) enthält, bevorzugt im Bereich von 190 g/mol bis 300 g/mol, weiter bevorzugt mit einem Molgewicht im Bereich von 195 g/mol bis 290 g/mol, und am meisten bevorzugt im Bereich 200 bis 275 g/mol.
und/oder
(C) (ii) Dipropylenglycol, Diethylphtalat, Triethylcitrat, Isopropylmyristat und Triacetin, wobei die Gesamtmenge des Bestandteils (C) (ii) bis zu 80 Gew.-%, bevorzugt von 0,5 bis 60 Gew.-%, bevorzugt von 1 bis 50 Gew.-%, weiter bevorzugt von 5 bis 40 Gew.-%, jeweils bezogen auf das Gesamtgewicht des Bestandteils (B) beträgt,
und/oder
(D) ein oder mehrere Di- oder Triole mit 3 bis 12 C-Atomen in einer Gesamtmenge von 0,2 bis 20 Gew.-%, bevorzugt von 0,5 bis 10 Gew.-%, weiter bevorzugt von 1 bis 5 Gew.-%,
jeweils bezogen auf das Gesamtgewicht der Zusammensetzung bzw. des Produktes.

10. Verfahren zur
- Reduzierung der Verdampfung von Riechstoffen in einer Riechstoffzusammensetzung, insbesondere unmittelbar nach der Applikation,
- zeitlichen Stabilisierung des Geruchsprofils einer Riechstoffzusammensetzung,
- Erhöhung oder zeitlichen Stabilisierung des geruchlichen Impacts (der wahrgenommenen Geruchsstärke) einer Riechstoffzusammensetzung,
- Verlängerung der Haftung von Riechstoffen einer Riechstoffzusammensetzung, insbesondere der Kopfnote, auf Haut und/oder Haar, insbesondere auf menschlicher Haut,
und/oder
- Vermittlung oder Verstärkung eines angenehmen Hautgefühls einer (ethanolischen) Riechstoffzusammensetzung,
mit folgendem Schritt:
Mischen von
(A) Neopentylglycoldiisononanoat, vorzugsweise in einer Bestandteil (B) fixierenden Menge,
und
(B) einem oder mehreren Riechstoffen,
wobei Bestandteil (B)
(B) (i) einen, 2, 3, 4, 5 oder mehr Riechstoffe mit einem Molgewicht im Bereich von 100 g/mol bis 175 g/mol (Kopfnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol.

11. Verfahren zur
- Vermittlung, Verstärkung oder Modifizierung eines Geruchs auf der (menschlichen) Haut,
- Verlängerung der Haftung von Riechstoffen einer Riechstoffzusammensetzung, insbesondere der Kopfnote, auf Haut und/oder Haar, insbesondere auf menschlicher Haut,
und/oder
- Vermittlung oder Verstärkung eines angenehmen Hautgefühls einer (ethanolischen) Riechstoffzusammensetzung,
mit folgendem Schritt:
- Applizieren einer Zusammensetzung nach einem der Ansprüche 1 bis 7 oder 9 oder eines Produktes nach Anspruch 8 oder 9 auf (menschliche) Haut und/oder (menschliches) Haar.

12. Verfahren nach Anspruch 10 oder 11, **dadurch gekennzeichnet, dass** die Riechstoffzusammensetzung einen, mehrere oder sämtliche Kopfnoten-Riechstoffe enthält ausgewählt aus der Gruppe bestehend aus:
(B) (i) n-Heptanol, trans-9-Decen-1-ol, Campher, alpha-Pinen, beta-Pinen, gamma-Terpinen, alpha-Phellandren, alpha-Terpineol, Borneol, Limonen, 6-Methyl-5-hepten-2-on, n-Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Benzaldehyd, Linalool, Tetrahydrolinalool, Tetrahydrogeraniol, Citral, Neral, Geranial, Benzylalkohol, Benzylacetat, Methylcinnamat, p-Anisaldehyd, Menthol, Isoamylacetat, Isoamylbutyrat, cis-3-Hexenylacetat, trans-3-Hexenylacetat, Hexylacetat, Butylbutyrat, Citronellol, Nerol, Geraniol, 2-Phenylethylalkohol, Methylbenzoat, Methylanthranilat, Styrallylacetat, 2,4-Dimethyl-3-cyclohexencarboxaldehyd (Vertocitral), Acetophenon, p-Methylacetophenon, Zimtalkohol, Zimtaldehyd, 3-Phenylpropanol (Dihydrozimtalkohol), 2-Methyl-1-phenyl-2-propanol (alpha,alpha-Dimethylphenethylcarbinol), Anethol, Anisalkohol, Heliotropin, Acetanisol (p-Methoxyacetophenon) und Agrunitril (3,7-Dimethyl-6-octen-1-nitril).

13. Verwendung von Neopentylglycoldiisononanoat als Fixateur für Parfümöle bzw. als Fixateur für (Kopfnoten-) Riechstoffe, wobei vorzugsweise die Kopfnoten-Riechstoffe gewählt sind aus der Liste bestehend aus:
(B) (i) n-Heptanol, trans-9-Decen-1-ol, Campher, alpha-Pinen, beta-Pinen, gamma-Terpinen, alpha-Phellandren, alpha-Terpineol, Borneol, Limonen, 6-Methyl-5-hepten-2-on, n-Octanal, Eucalyptol (1,8-Cineol), Rosenoxid, 3-Hexenol, Dihydromyrcenol (2,6-Dimethyl-7-octen-2-ol), Menthon, Isomenthon, 2,6-Dimethyl-5-hepten-1-al (Melonal), 3-Hexenylmethylcarbonat, Benzaldehyd, Linalool, Tetrahydrolinalool, Tetrahydrogeraniol, Citral, Neral, Geranial, Benzylalkohol, Benzylacetat, Methylcinnamat, p-Anisaldehyd, Menthol, Isoamylacetat, Isoamylbutyrat, cis-3-Hexenylacetat, trans-3-Hexenylacetat, Hexylacetat, Butylbutyrat, Citronellol, Nerol, Geraniol, 2-Phenylethylalkohol, Methylbenzoat, Methylanthranilat, Styrallylacetat, 2,4-Dimethyl-3-cyclohexencarboxaldehyd (Vertocitral), Acetophenon, p-Methylacetophenon, Zimtalkohol, Zimtaldehyd, 3-Phenylpropanol (Dihydrozimtalkohol), 2-Methyl-1-phenyl-2-propanol (alpha,alpha-Dimethylphenethylcarbinol), Anethol, Anisalkohol, Heliotropin, Acetanisol (p-Methoxyacetophenon) und Agrunitril (3,7-Dimethyl-6-octen-1-nitril).

14. Verwendung von Neopentylglycoldiisononanoat als Mittel zur
- Reduzierung der Verdampfung von Riechstoffen in einer Riechstoffzusammensetzung, insbesondere unmittelbar nach der Applikation;
- zeitlichen Stabilisierung des Geruchsprofils einer Riechstoffzusammensetzung;
- Erhöhung oder zeitlichen Stabilisierung des geruchlichen Impacts (der wahrgenommenen Geruchsstärke) einer Riechstoffzusammensetzung;
- Verlängerung der Haftung von Riechstoffen einer Riechstoffzusammensetzung, insbesondere der Kopfnote, auf Haut und/oder Haar, insbesondere auf menschlicher Haut;
und/oder
- Vermittlung oder Verstärkung eines angenehmen Hautgefühls einer Riechstoffzusammensetzung.

15. Riechstoff-Fixierungsmittel - Kit, umfassend
eine erste Komponente umfassend oder bestehend aus
(A) Neopentylglycoldiisononanoat,
und
eine zweite Komponente umfassend oder bestehend aus
(B) eine oder mehreren Riechstoffen, vorzugsweise einer Riechstoffmischung umfassend 2, 3, 4, 5, 6, 7, 8, 9, 10 oder mehr Riechstoffe,
wobei Bestandteil (B)
(B) (i) einen, 2, 3, 4, 5 oder mehr Riechstoffe mit einem Molgewicht im Bereich von 100 g/mol bis 175 g/mol (Kopfnote) enthält, vorzugsweise mit einem Molgewicht im Bereich von 110 g/mol bis 165 g/mol, bevorzugt im Bereich von 115 g/mol bis 160 g/mol, besonders bevorzugt im Bereich von 120 g/mol bis 155 g/mol,
wobei vorzugsweise Bestandteil (B) gewählt ist, wie in einem der Ansprüche 3 bis 5 definiert.
